# EUROPEAN PATENT SPECIFICATION

(11) **EP 3 883 374 B1**
(45) Date of publication and mention of the grant of the patent: **26.10.2022**
(21) Application number: 19813186.4
(22) Date of filing: 21.11.2019
(51) Int. Cl.: A01K 67/033, B04C 5/14, B04C 5/18, B04C 9/00, B04C 5/04

(54) **CYCLONE SEPARATION SYSTEM**
ZYKLONABSCHEIDUNGSSYSTEM
SYSTÈME DE SÉPARATION À CYCLONE

(30) Priority: 23.11.2018 NL 2022057; 21.12.2018 WO PCT/NL2018/050867; 06.06.2019 US 201962857842 P; 14.06.2019 NL 2023315
(43) Date of publication of application: 29.09.2021
(73) Proprietor: Protix B.V., 5107 NC Dongen (NL)
(72) Inventor: VAN KILSDONK, Jaap, 5509 KG Veldhoven (NL); SCHMITT, Eric Holland, 2060 Antwerpen (BE); JACOBS, Ralf Henricus Wilhelmina, 5643 LT Eindhoven (NL); SIMONS, Henricus Petrus Johannes, 5223 KL Den Bosch (NL); JANSEN, Maurits Petrus Maria, 4854 CA Bavel (NL); TOLLENAAR, Ward, 5101 AR Dongen (NL); HULSEBOS, Hubertus Lourentius, 5046 GE Tilburg (NL)
(74) Representative: Nederlandsch Octrooibureau
(86) International application number: PCT/NL2019/050767
(87) International publication number: WO 2020/106150

(56) References cited:
- FR-A- 1 130 633
- GB-A- 429 028
- RU-C1- 2 336 696
- US-A- 4 853 003
- US-A- 5 594 654
- US-A1- 2005 242 007
- US-A1- 2018 049 414
- US-B1- 10 051 845

## Description

### Field of the invention

The present invention relates to a cyclone separation system for separating live insects from an air stream. The invention also relates to the cyclone separation system provided with an insects transport device for provision of live insects into the cyclone separation system. In a further aspect the present invention relates to a method of separating live insects from an air stream, and in particular a method of providing batches of live insects.

### Background art

US patent publication US 2018 / 0049418 A1 discloses a variable-scale computer operated Insect Production Superstructure Systems (IPSS) for the production of insects for human and animal consumption, and for the extraction and use of lipids for applications involving medicine, nanotechnology, consumer products, and chemical production with minimal water, feedstock, and environmental impact. An IPSS may comprise modules including feed stock mixing, enhanced feedstock splitting, insect feeding, insect breeding, insect collection, insect grinding, pathogen removal, multifunctional flour mixing, and lipid extraction. In an embodiment, an insect feeding module is in fluid communication with an insect evacuation module comprising separator that may be a cyclone for separating insects from a gas.

U.S. patent publication US 5,594,654 discloses an automated system developed to count and package beneficial insect larvae or eggs and includes a funnel-shaped container which sits in the top portion of a sensor head and a turntable with multiple containers located below the sensor head, for collecting larvae or eggs as they drop through the sensor head. The system accurately records the number and time stamps each insect larva or egg detection as they drop through a sensor head.

US 2018/049414 A1 is related to variable-scale, modular, easily manufacturable, energy efficient, reliable, and computer operated Insect Production Superstructure Systems (IPSS), which may be used to produce insects for human and animal consumption, and for the extraction and use of lipids for applications involving medicine, nanotechnology, consumer products, and chemical production with minimal water, feedstock, and environmental impact.

GB 429 028 A is related to centrifugal separators for separating and collecting dust or other solid particles from air and gases, and of the class in which the dust-laden air or gas is propelled into a vessel in such manner as to create therein a vortical motion.

RU2336696 is related to an installation unit for the cultivation of larvae of synanthropic flies. The installation unit includes body with transverse slot mounted on height adjustable supports for horizontality of unit, storage tank for feeding slop for maggots, feeding slop feeder to body, premature migration preventive fan averting maggot migration from casing and removal after growth period is complete, and tank for separated maggots.

### Summary of the invention

The present invention aims to provide a cyclone separation system for separating live insects from an air stream, such as neonate larvae, wherein the cyclone separation system allows for efficient and reliable batch wise discharge of live insects from the cyclone separation system whilst keeping the live insects alive and preventing that the live insects stick or adhere to internal walls of the cyclone separation system. The cyclone separation system is ideally suited for being integrated in an automated live insect processing facility.

According to the present invention, a cyclone separation system of the type defined in claim 1 is provided, wherein the cyclone separation system comprises a main cyclone chamber having a top chamber part and a conical shaped bottom chamber part. The top chamber part is connected to one or more intake channels each of which is arranged for connection to a primary air source providing an air stream laden with live insects, and wherein the bottom chamber part is connected to a discharge nozzle that comprises a discharge end having a main discharge conduit for discharging the (separated) live insects from the cyclone separation system. The discharge end of the discharge nozzle comprises an air injection member that is arranged for connection to a secondary air source and wherein the air injection member is configured to inject air back into the discharge nozzle for stopping the discharge of the separated live insects. Moreover, a method of providing batches of live insects according to claim 19 is also provided.

According to the present invention, the air injection member of the discharge end is configured for injecting air back into the discharge nozzle, *i.e.* in upstream direction, so that separated live insects in the discharge nozzle and moving in a direction toward the discharge end, *i.e.* in downstream direction, can be stopped and air suspended/cushioned by the injected air. Through injection of back/upstream flowing air into the discharge nozzle the discharge of live insects can be stopped and as such the air injection member acts like a controllable air valve. Furthermore, the injected air allows live insects to be air suspended/cushioned, e.g. pushed in upstream direction, thereby preventing the live insects from sticking to inner walls of the discharge nozzle and a such prevent clump formation of live insects that could potentially block the discharge nozzle.

Another advantage of the air injection member is that intermittent, time limited air injections back into the discharge nozzle can be performed, thereby achieving intermittent discharge of live insects between two successive air injections. The time interval between two successive air injections then determines a batch of discharged live insects that can be collected and transferred for further processing. Transferring such a collected batch of live insects is achieved during such a time limited air injection.

In an embodiment, the air injection member of the discharge end comprises an air chamber and an air injection conduit (i.e. a first air injection conduit) fluidly (e.g. gaseous) connecting the air chamber and the main discharge conduit of the discharge end. The air injection conduit is configured to provide an injected air flow in a direction back into the discharge nozzle when air is pushed through the air injection conduit. In this embodiment the air injection conduit allows for the injected air flow to be directed into the discharge nozzle in upstream fashion such that live insects are effectively suspended in air, thereby stopping the discharge. For example, in an embodiment the air injection conduit is arranged at an injection angle smaller than 60° degrees with respect to a longitudinal axis of the discharge nozzle, so that the air flow being injected does indeed move in a direction back into the discharge nozzle.

According to the present invention, it is possible to utilize a plurality of air injection conduits that are configured to provide an injected air flow in a direction back into the discharge nozzle. For example, the air injection member of the discharge end may comprise a further or second air chamber and a further or second air injection conduit fluidly (e.g. gaseous) connecting the further/second air chamber and the main discharge conduit of the discharge end, wherein the further/second air injection conduit is arranged to provide a further/second injected air flow in a direction back into the discharge nozzle. Like the air injection conduit (i.e. the first air injection conduit), the further/second air injection conduit allows for a further/second injected air flow to be directed into the discharge nozzle in upstream fashion such that live insects are effectively air suspended or air cushioned for further stopping the discharge of live insects.
in an embodiment the further/second air injection conduit is arranged at a further/second injection angle smaller than 60° degrees with respect to the longitudinal axis of the discharge nozzle, so that the further/second air flow being injected through the further/second air injection conduit does indeed move in a direction into the discharge nozzle.

Utilizing a plurality of injection conduits for injecting air into the main discharge conduit allows for further optimization of injected air flow. For example, in an embodiment the aforementioned first and further/second air injection conduits may be arranged on opposite sides of the main discharge conduit. This embodiment then allows two separate air flows to be injected back into the discharge nozzle for an overall improved flow distribution of injected air throughout the discharge nozzle. This in turn allows for improved distributed air suspension/cushioning of live insects for stopping the discharge thereof.

Since the air injection member is configured for connection to a secondary air source, it may be advantageous to minimize and simplify the number of physical connections of the secondary air source to the air injection member. To that end an embodiment may be considered wherein the first and second air chambers are arranged on opposite sides of the main discharge conduit and are fluidly (e.g. gaseous) connected to one another. That is, in this embodiment the first and second air chambers are fluidly (e.g. gaseous) coupled and may be envisaged as forming a single air chamber circumferentially encircling the main discharge conduit. Since the first and second air chambers effectively form a single air chamber, it is possible to utilize a single air inlet configured to connect to the secondary air source and wherein the single air inlet also fluidly connects to the interconnected first and second air chambers.

### Short description of drawings

The present invention will be discussed in more detail below, with reference to the attached drawings, in which
Figure 1 shows a schematic view of a cyclone separation system according to an embodiment of the present invention;
Figure 2 shows a three dimensional view of a discharge nozzle according to an embodiment of the present invention;
Figure 3A shows a first cross section of a discharge nozzle according to an embodiment of the present invention;
Figure 3B shows a first cross section of a discharge end of a discharge nozzle according to an embodiment of the present invention;
Figure 4A shows a second cross section of a discharge nozzle according to an embodiment of the present invention;
Figure 4B shows a second cross section of a discharge end of a discharge nozzle according to an embodiment of the present invention;
Figure 5A shows a three dimensional view of a main discharge conduit with a first air injection conduit according to an embodiment of the present invention;
Figure 5B shows a three dimensional view of a main discharge conduit with a second air injection conduit according to an embodiment of the present invention; and
Figure 6 shows a schematic view of a camera based counting system arranged at a discharge end of a discharge nozzle according to an embodiment of the present invention;
Figure 7 shows a third cross section of a discharge nozzle according to an embodiment of the present invention;
Figure 8 shows a fourth cross section of a discharge nozzle according to an embodiment of the present invention;
Figure 9 shows a top view of an intake end of a discharge nozzle according to an embodiment of the present invention;
Figure 10 shows a fifth cross section of a discharge nozzle according to an embodiment of the present invention;
Figure 11 shows another top view of an intake end of a discharge nozzle according to an embodiment of the present invention;
Figure 12 shows a cross section of a discharge nozzle and a container arranged underneath the discharge nozzle according to an embodiment of the present invention;
Figure 13 shows another schematic view of a camera based counting system arranged at a discharge end of a discharge nozzle according to an embodiment of the present invention;
Figure 14 displays an overview of an embodiment of the invention, showing an insects transport device 1 in an air-conditioned ('climatized') room 900. The insects transport device is tilted relative to the horizontal over an angle α (alpha). Further, an insect discharge member 11 is indicated, provided with a camera 8 and a lamp 9;
Figure 15 displays an overview of an insects transport device 1 of the invention comprising a thermally insulated casing 5 and a gas guiding unit 12 that provides a smooth longitudinal path for a laminar flow of gas, and further displays the distal end 15 of the gas guiding unit which receives the gas discharge members 20, 20' through an opening 17 in the casing 5;
Figure 16 displays a detailed side view of an insects transport device 1 of the invention where the proximal end of the gas guiding unit 12' ends and where the insect discharge member (See also 11 in Figure 15) is located and coupled to said proximal end;
Figure 17 displays an inside view of an insects transport device of the invention. Shown are longitudinal gas transport members 12', 12" which are connected imbricatedly at positions 21, 22 and 21', 22'. Where two consecutive gas transport members are coupled imbricatedly, a gas discharge member (See 20, 20' in Figure 15 and 114', 114", 114‴ in Figure 18) is positioned at the location where said gas transport members overlap, said gas discharge member provided with openings 23, 23' for discharging gas;
Figure 18 displays an overview of another embodiment of the invention, showing an insects transport device 100 comprising a live insects receiving portion that is built up by a gas guiding unit 112 comprising side walls 113 tilted at an obtuse angle relative to the top surface of the gas guiding members. The insects transport device of the embodiment comprises a thermally insulated casing 105, said casing having a top side 102 optionally made at least in part from a transparent material 125 such as a plate made of glass;

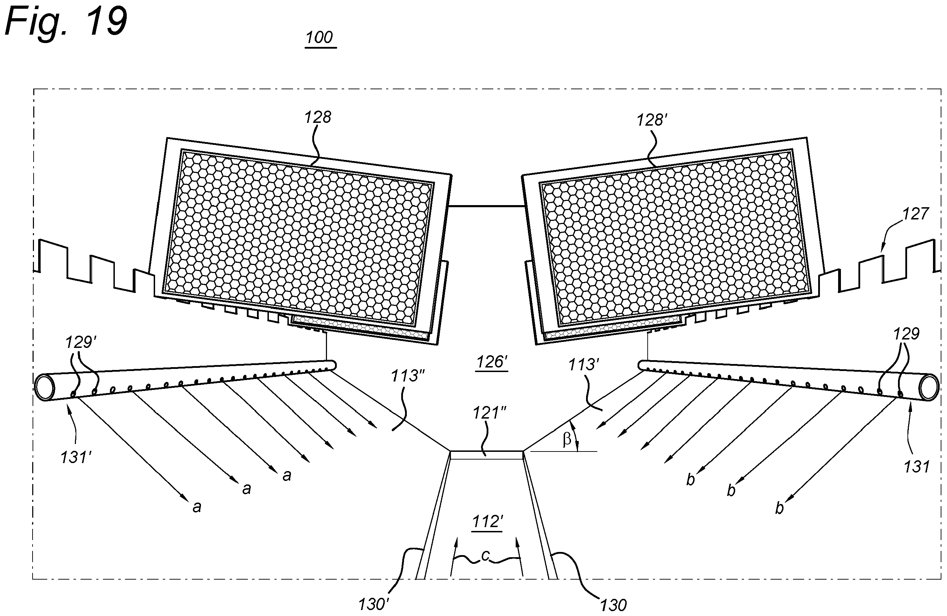

Figure 19 displays a part of a live insects receiving portion of an insects transport device 100 of the invention, the live insects receiving portion being built up by a gas guiding unit 112' comprising side walls 113'and 113" tilted at an obtuse angle relative to the top surface of the gas guiding members. Further displayed are the proximal end 121" of the live insects guiding unit 112' and the further gas discharge members 131 and 131' located at the top side of the side walls, and the feeder arrangement 127 located above the live insects receiving portion of the top surface of the gas guiding unit;

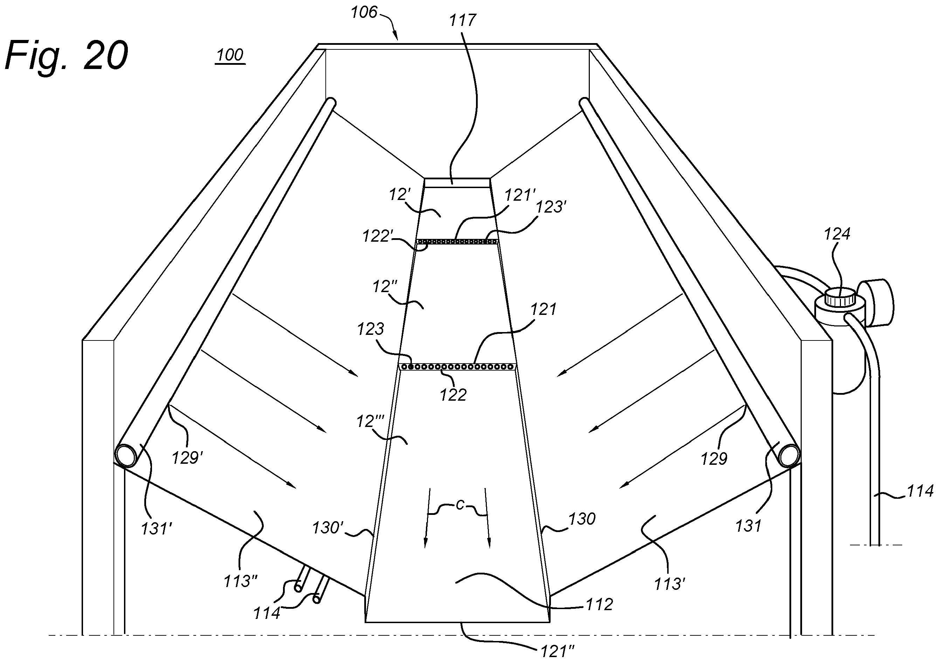

Figure 20 displays a view of an insects transport device 100 of the invention along the longitudinal gas guiding units in the direction towards the first gas discharge member located at opening 117. Consecutive gas guiding units are connected imbricatedly and at positions where the gas guiding units overlap imbricatedly further gas discharge members are located for reinforcing the first laminar flow of gas. The live insects receiving portion is shown and is built up by a gas guiding unit 112' comprising side walls 113' and 113" tilted at an obtuse angle relative to the top surface of the gas guiding members. Further displayed are the distal end of the live insects guiding unit and the further gas discharge members 131' and 131 located at the top side of the side walls 113" and 131', respectively;
Figure 21 depicts an insects transport device 100 comprising a gas guiding unit 112 and arched convex side walls 113', 113" arranged there along according to an embodiment of the present invention;
Figure 22 depicts an insects transport device 100 comprising a cover member 132 arranged over and along a gas guiding unit 112 according to an embodiment of the present invention;
Figure 23 shows a thermally insulated casing 5 of an insects transport device 100 according to an embodiment of the present invention, the insects transport device comprising a reservoir 128, the reservoir being an ovisite;
Figure 24 shows a three dimensional view of a live insect discharge member 11 according to an embodiment of the present invention;
Figure 25 shows a cross sectional view of a live insect discharge member 11 according to an embodiment of the present invention;
Figure 26 shows a schematic view of a cyclone separation system 1K further provided with an insects transport device 100 connected to the live insect discharge member 11, according to an embodiment of the present invention;
Figure 27A shows a top view of the cyclone separation system 1K, comprised by the insects transport device of the invention, showing laminar slats that are openable under control of a control unit;
Figure 27B shows a perspective top/side view of the cyclone separation system 1K, comprised by the insects transport device of the invention, showing laminar slats in the top portion 148' of the system 1K;
Figure 27C shows a side view of part of the cyclone separation system 1K;
Figure 28A shows a reservoir 128a, consisting of a cage for live insects such as mite, the cage comprising side walls and a bottom floor comprising openings for passage of live insects;

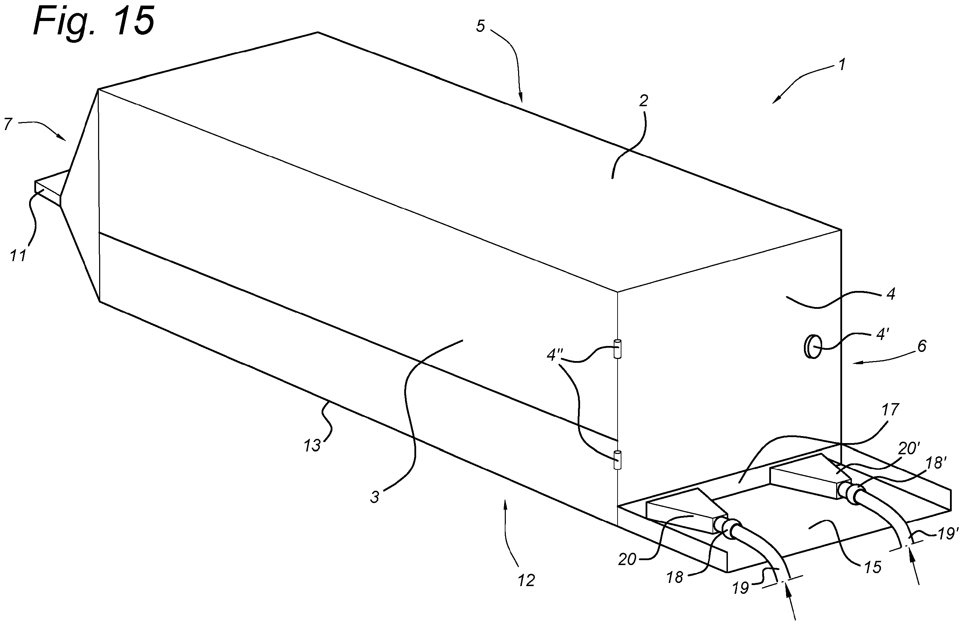

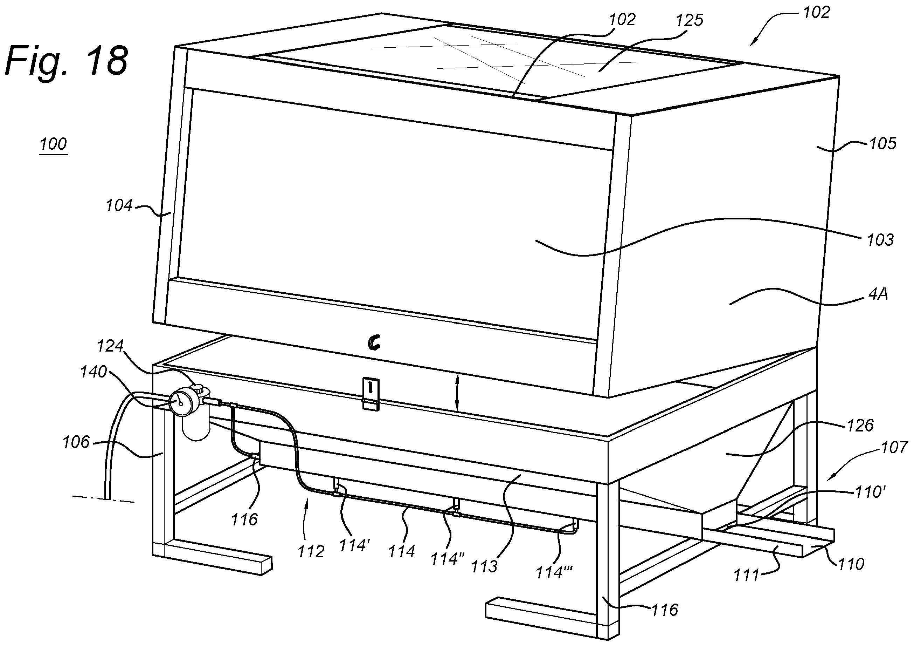

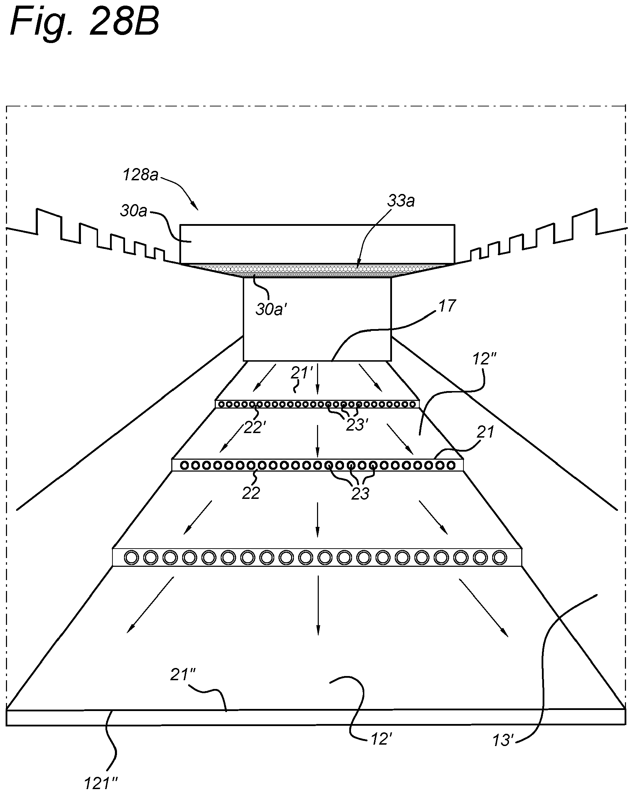

Figure 28B displays an inside view of an insects transport device of the invention. Shown are longitudinal gas transport members 12', 12" which are connected imbricatedly at positions 21, 22 and 21', 22'. Where two consecutive gas transport members are coupled imbricatedly, a gas discharge member (See 20, 20' in Figure 15 and 114', 114", 114‴ in Figure 18) is positioned at the location where said gas transport members overlap, said gas discharge member provided with openings 23, 23' fordischarging gas. The insects transport device comprises a reservoir 128a, the reservoir being a cage for live insects, the cage comprising side walls and a bottom floor comprising openings for passage of live insects;
Figure 28C shows a thermally insulated casing 5 of an insects transport device 100 according to an embodiment of the present invention, the insects transport device comprising a reservoir 128a, the reservoir being a cage for live insects, the cage comprising side walls and a bottom floor comprising openings for passage of live insects, the casing comprising a secondary top wall 2a defining a volume 135;
Figure 29A displays an insect discharge member 11a coupled to a tube 11b, the tube 11b connected to an air amplifier unit 142';
Figure 29B displays a cross-sectional side view of the insect discharge member 11a connected to tube 11b;
Figure 29C shows a cross-sectional side view of air amplifier unit 142' fluidly connected to tube 11b, which is connected at its proximal end to the insect discharge member 11a as displayed in Figure 29B;
Figure 29D shows a schematic view of an insects transport device 100 further provided with a cyclone separation system 1Kfluidly connected to the live insect discharge member 11a via tubing 11b and air amplifier unit 142', according to an embodiment of the present invention;
Figure 30A displays an exploded view of an insects transport device 1, 100, showing the side walls and top wall of the casing 5, 105, said side walls and top wall provided with a layer of thermally insulating material 301-305, wherein the side wall 4 is an openable door 4;
Figure 30B displays an insects transport device 1, 100 provided with casing 5, 105, wherein said casing comprises thermally insulated side walls and a thermally insulated top wall. For clarity the front side wall 4 is not shown;
Figure 30C displays an insects transport device 1, 100 provided with casing 5, 105, wherein said casing comprises thermally insulated side walls and a thermally insulated top wall, according to an embodiment of the invention;
Figure 31 depicts an insects transport device 100 comprising a gas guiding unit 112 and arched convex side walls 113', 113" arranged there along according to an embodiment of the present invention;
Figure 32 depicts an insects transport device 100 comprising a cover member 132 arranged over and along a gas guiding unit 112, further comprising a gas guiding unit 112 and arched convex side walls 113', 113" arranged there along and air slits 607a and 607b arranged along the top side of the arched convex side walls, according to an embodiment of the present invention;
Figure 33 shows a schematic view of a cyclone separation system 1K further provided with an insects transport device 100 connected to the live insect discharge member 11, according to an embodiment of the present invention;
Figure 34 shows a thermally insulated casing 5 of an insects transport device 100 according to an embodiment of the present invention, the insects transport device comprising a reservoir 128, the reservoir being an ovisite;
Figure 35 displays an insect discharge member 11a coupled to a tube 11b, the tube 11b connected to an air amplifier unit 142' comprising a driver (a fan) 803, an air inlet for air 802, a sensor 801 for sensing air humidity and temperature;
Figure 36 shows a schematic view of a cyclone separation system 1K further provided with an insects transport device 100 connected to the live insect discharge member 11, wherein the opening 707 in top chamber part 3K of the cyclone separation system 1K is essentially at the same height relative to the horizontal as the proximal end 121" of gas guiding unit 112, the cyclone separation system 1K further provided with sensor 700 for sensing air humidity and temperature of air inside the cyclone separation system 1K, according to an embodiment of the present invention; and wherein
Figure 37 shows the cyclone separation system 1K comprising four insect transport devices 100 connected to the cyclone separation system 1K through connectors 707a-707d, wherein the openings 707a-d in top chamber part 3K of the cyclone separation system 1K are essentially at the same height relative to the horizontal as the proximal ends 121" of gas guiding units of the four insect transport devices 100.

### Description of embodiments

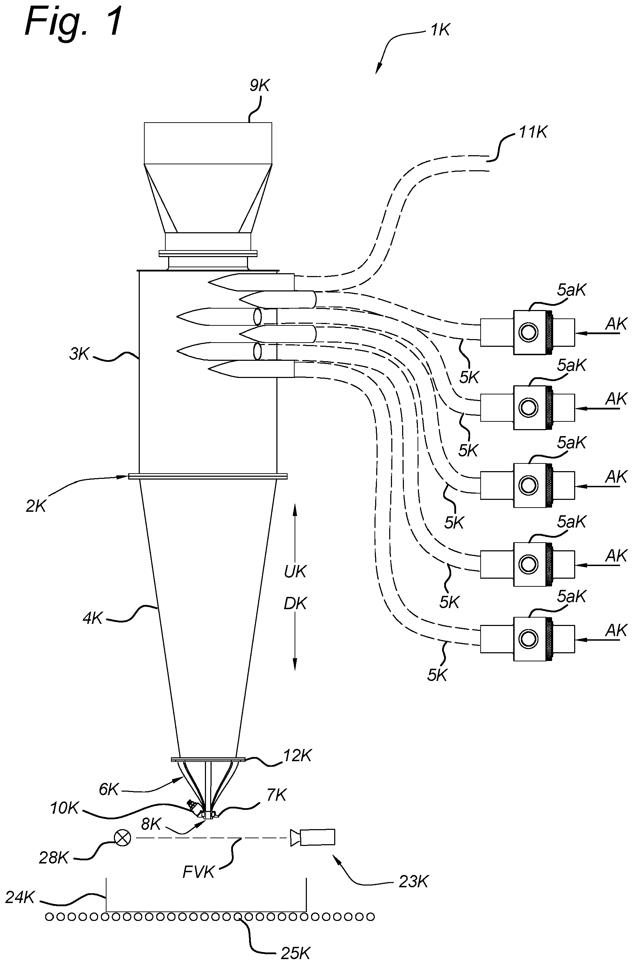

Figure 1 depicts a schematic view of a cyclone separation system 1K for separating and batch wise discharging live insects carried by one more air streams AK. The cyclone separation system 1 K comprises a main cyclone chamber 2K having a top chamber part 3K and a conical shaped bottom chamber part 4K, *e.g.* a hopper. The top chamber part 3K is connected to one or more intake channels 5K each of which is arranged for connection to a primary air source (not shown) providing an air stream AK comprising live insects. The live insects under consideration may be viewed as granular matter comprising various types of larvae, such as neonate larvae. The bottom chamber part 4K is connected to a discharge nozzle 6K comprising a discharge end 7K which has a main discharge conduit 8K for discharging the separated live insects from the cyclone separation system 1K.

As the skilled person will understand, in operation the one or more intake channels 5K carrying the air streams AK induce a main vortex in the top chamber part 3K that allows centrifugal separation of the live insects from the (combined) air streams AK. The separated live insects then follow conical inner walls of the bottom chamber part 4K toward the discharge nozzle 6K. Due to the conical shaped bottom chamber part 4K, an ascending inner vortex of "clean" air is generated that exits the top chamber part 3K through an air exit 9K arranged thereon.

As further depicted, the discharge end 7K of the discharge nozzle 6K comprises an air injection member 10K for connection to a secondary air source (not shown) and wherein the air injection member 10K is configured to inject air back into the discharge nozzle 6K.

According to the present invention, the air injection member 10K of the discharge end 7K is configured to inject air back into the discharge nozzle 6K, *i.e.* in an upstream direction "UK", so that separated live insects moving downstream into the discharge end 7K, *i.e.* in downstream direction "DK", can be stopped from discharging through suspension by the injected air. By virtue of injection of backward or upstream flowing air into the discharge nozzle 6K, the discharge of live insects can be stopped and as such the air injection member 10K acts as a controllable air valve allowing the main discharge conduit 8K to be opened or closed through a "wall" of upstream flowing air.

Furthermore, as the injected air effectively cushions the live insects in air, prolonged contact of live insects with inner walls of the discharge nozzle 6K is prevented. This ensures that live insects are less prone to stick to inner walls of the discharge nozzle and a such prevent clump formation therein.

As will be discussed in further detail below, another advantage of the air injection member 10K is that intermittent, time limited air injections back into the discharge nozzle 6K can be utilised for intermittent discharge of separated live insects between two successive air injections when the cyclone separation system 1K is in operation. The time interval between two successive air injections then determines a batch of discharged live insects that can be collected and transferred for further processing. Transferring a collected batch of live insects can be achieved during a subsequent air injection by the air injection member 10K.

In an embodiment, the top chamber part 3K may be further connected to an auxiliary intake channel 11K arranged to receive additional air, called "pilot air", to further optimize vortex generation within the top chamber part 3K.

To maintain sufficient pressure and air flow within the one or more intake channels 5K, an embodiment may be provided wherein each of the intake channels 5K comprises an air amplifier unit 5aK, which is configured to provide a supplementary air stream to the air stream AK in a flow direction thereof.

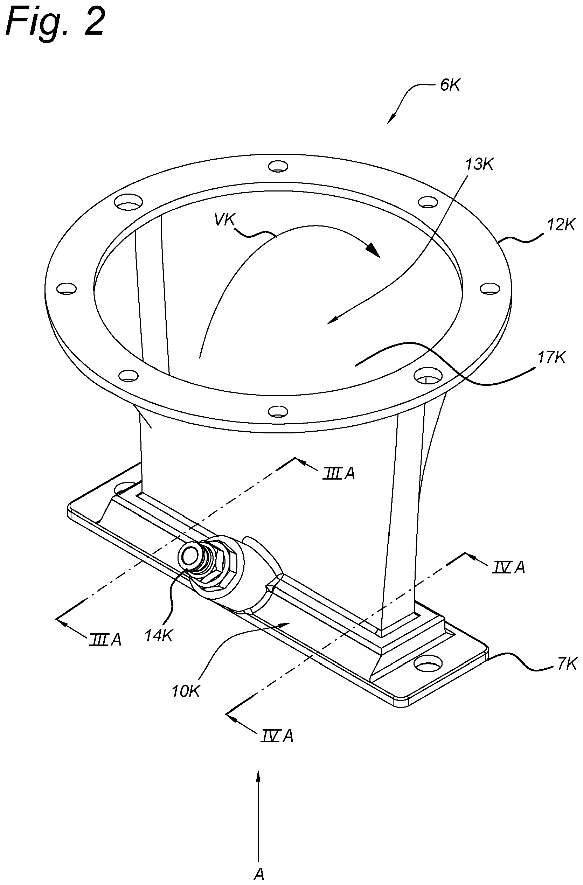

Figure 2 shows a three dimensional view of a discharge nozzle 6K according to an embodiment of the present invention, wherein the discharge nozzle 6K comprises the aforementioned discharge end 7K but may further comprise an intake end 12K that may be utilized to connect the discharge nozzle 6K, e.g. through a bolt on flange or a quick-release flange connection, to the bottom chamber part 4K. As shown in the depicted embodiment, the intake end 12K may be circular, matching a circular shape of the bottom chamber part 4K, and wherein the discharge end 7K may have a substantially rectangular shape with a substantially rectangular main discharge conduit (not visible). Generally, the discharge nozzle 6K provides a funnel shaped passage 13K allowing separated live insects to converge to the main discharge conduit of the discharge end 7K.

It is noted that an embodiment is conceivable wherein the bottom chamber part 4K and the discharge nozzle 6K are integrated as a single piece to reduce the number of ridges at which live insects could potentially stick and clump together.

The air injection member 10K may further comprise an air inlet 14K for connecting to the secondary air source and wherein the air inlet 14K is fluidly (gaseous) connected to the main discharge conduit 8K allow air injecting back into the discharge nozzle.

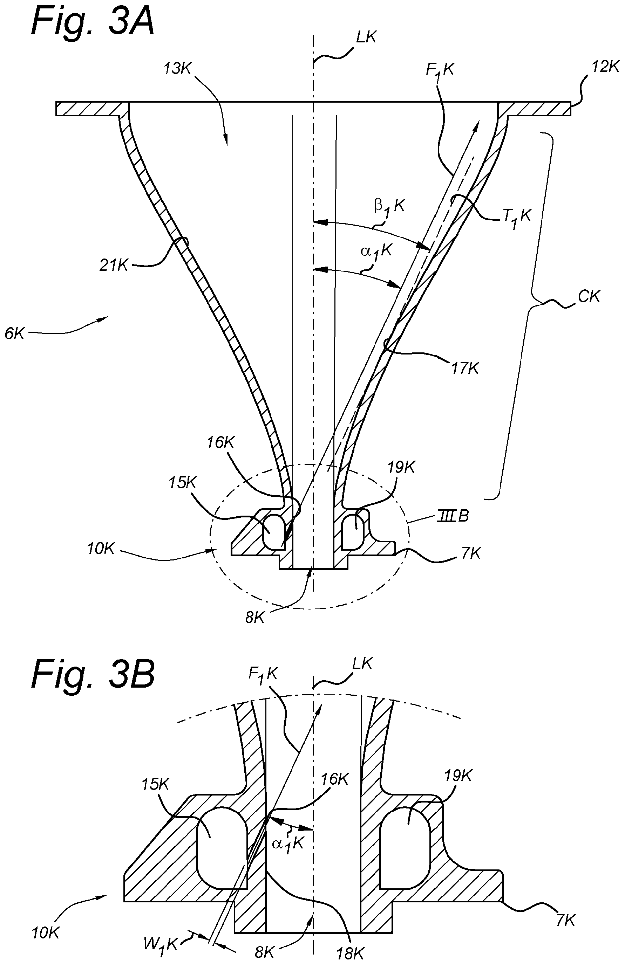

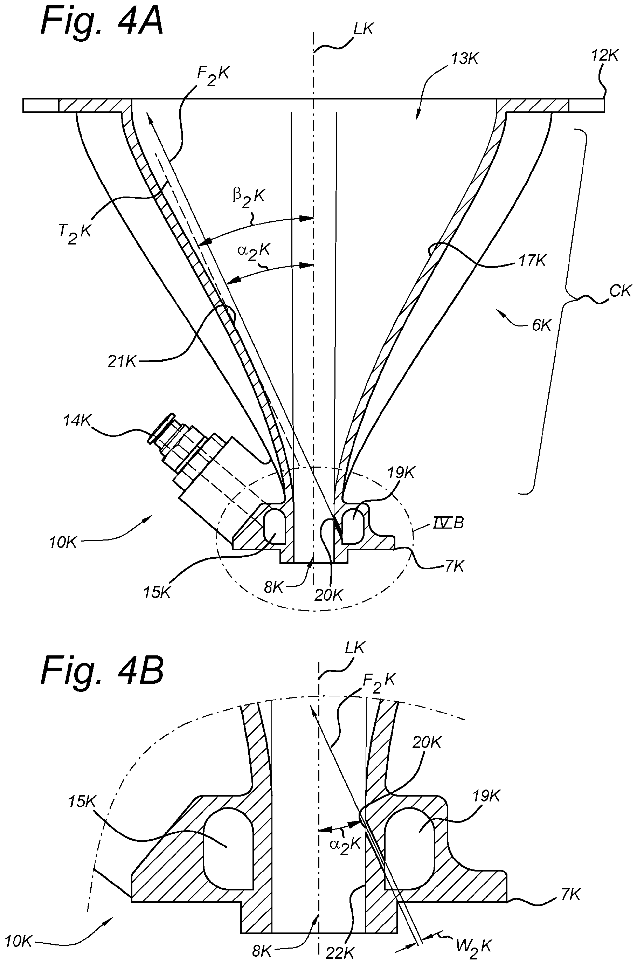

Note that Figure 2 further indicates a first cross sectional view "III A" and a second cross sectional view "IV A", which are depicted in Figure 3A and Figure 4A respectively.

In particular, Figure 3a shows the indicated first cross section "III A" of a discharge nozzle 6K according to an embodiment of the present invention. In the embodiment shown, the air injection member 10K comprises an air chamber 15K, *i.e.* a first air chamber 15K, and an air injection conduit 16K, i.e. a first air injection conduit 16K, fluidly (gaseous) connecting the first air chamber 15K and the main discharge conduit 8K of the discharge end 7K. As further shown, the first air injection conduit 16K is configured to provide an injected air flow F1K, *i.e.* a first injected air flow F1K, in a direction back into the discharge nozzle 6K. An advantage of the first air chamber 15K is that the location and orientation of the first air injection conduit 16K in the discharge end 7K can be chosen more freely to accommodate a specific design of the discharge nozzle 6K and particular shape and direction of the first injected air flow F1K, as long as the first air injection conduit 16K fluidly (gaseous) connects to the first air chamber 15K.

In an embodiment, the first air injection conduit 16K is arranged at an injection angle α₁K, *i.e.* a first injection angle α₁K, smaller than 60° degrees with respect to a longitudinal axis LK of the discharge nozzle 6K. The first injection angle α₁K less than 60° ensures that when air is being injected into the main discharge conduit 8K through the first air injection conduit 16K, that the first injected air flow F1K is directed into the discharge nozzle 6K for air suspension/cushioning the live insects and stop discharge thereof. In specific embodiments, the first injection angle α₁K may be 45° or less to ensure good back flow of injected air into the discharge nozzle 6K.

In advantageous embodiments, the first injected air flow F1K may engage an inner wall portion 17K, *i.e.* a first inner wall portion 17K, of the discharge nozzle 6K in parallel fashion as most live insects will descend into the bottom chamber part 4K and the discharge nozzle 6K along walls thereof. In an embodiment, the first inner wall portion 17K may be located somewhere halfway a converging section "CK" of the discharge nozzle 6K as sufficient convergence and compaction of live insects will have occurred at such a location for the first injected air flow F1K to be adequate for air suspension/cushioning separated live insects. It is noted that the skilled person will understand that the converging section "CK" may comprise various profiles of the first inner wall portion 17K and that the substantial parallel engagement of the first injected air flow F1K with the first inner wall portion 17K may occur closer or further away from the first air injection conduit 16K.

To allow forthe substantial parallel engagement between the first injected air flow F1K and the first inner wall portion 17K, an embodiment is provided wherein the discharge nozzle 6K comprises the first inner wall portion 17K which is arranged, e.g. at least locally, at a wall angle β₁K, *i.e.* a first wall angle β₁K, with respect to the longitudinal axis LK of the discharge nozzle 6K. The first injection angle α₁K of the first air injection conduit 16K is then substantially equal/aligned with the first wall angle β₁K. In this embodiment the first inner wall portion 17K is at least locally arranged at the first wall angle β₁K which substantially coincides with the first injection angle α₁K. This alignment of angles α₁K, β₁K allows the first injected air flow F1K to engage the first inner wall portion 17K in substantial parallel fashion for good air suspension/cushioning the separated live insects as most live insects descend into the discharge nozzle 6K along inner walls thereof, e.g. the first inner wall portion 17K. This embodiment may be further clarified by imagining a tangent line T₁K coinciding with the first inner wall portion 17K and wherein the tangent line T₁K is at the first inner wall angle β₁K. As depicted in Figure 3A, the first inner wall portion 17K may be (slightly) curved without significantly deviating from the tangent line T₁K. In an embodiment, the first injection angle α₁K of the first air injection conduit 16K is not smaller than the first wall angle β₁K to further ensure that substantial parallel engagement between the first injected air flow F1K and the first inner wall portion 17K is achieved.

Figure 3B shows the indicated cross section "III B" (see Figure 3A) of a discharge end 7K of a discharge nozzle 6K. In this embodiment it is clearly shown that the first air injection conduit 16K extends between and fluidly (gaseous) connects the first air chamber 15K and the main discharge conduit 8K. It is further shown that the first air injection conduit 16K may be a straight conduit extending between the first air chamber 15K and a discharge conduit wall portion 18K, *i.e.* a first discharge conduit wall portion 18K, to provide a shortest path from the first air chamber 15K to the main discharge conduit 8K for minimizing pressure loss and maximize the intensity of the first injected air flow F1K.

In an embodiment, the first air injection conduit 16K may have a width, i.e. a first width W₁K, between 0.2 mm and 1 mm to allow sufficiently strong air flow back into the discharge nozzle 6K for air suspension/cushioning separated live insects. It is noted that in this embodiment a smaller first width W₁K within this range will generally provide a faster first injected air flow F1K with less air usage compared to having a larger first width W₁K within this range forthe first air injection conduit 16K. Choosing smaller values for the first width W₁K will typically lead to reduced disturbance of the air flow within the discharge nozzle 6K.

Turning to Figure 4A, wherein the indicated second cross section "IV A" (see Figure 2) of a discharge nozzle 6K is depicted. In the embodiment shown, the air injection member 10K may comprise a further air chamber 19K, *i.e.* a second air chamber 19K, and a further air injection conduit 20K, *i.e.* a second air injection conduit 20K, fluidly (gaseous) connecting the second air chamber 19K and the main discharge conduit 8K. The second air injection conduit 20K is then arranged to provide a further injected air flow F2K, *i.e.* a second injected air flow F2K, in a direction back into the discharge nozzle 6K. As with the first air chamber 15K, an advantage of the second air chamber 19K is that the location and orientation of the second air injection conduit 20K can be chosen more freely to accommodate a specific design of the discharge nozzle 6K and a particular shape and direction of the second injected air flow F2K as long as the second air injection conduit 20K fluidly (gaseous) connects to the second air chamber 19K.

In an embodiment, the second air injection conduit 20K is arranged at a further injection angle α₂K, *i.e.* a second injection angle α₂K, smaller than 60° degrees with respect to a longitudinal axis LK of the discharge nozzle 6K. Providing a second injection angle α₂K less than 60° ensures that when air is being injected into the main discharge conduit 8K through the second air injection conduit 20K, that the second injected air flow F2K is primarily directed into the discharge nozzle 6K for air suspension/cushioning the live insects and stop discharge thereof. In specific embodiments, the second injection angle α₂K may be 45° or less to ensure good back flow of injected air into the discharge nozzle 6K.

In advantageous embodiments, the second injected airflow F2K may engage a further inner wall portion 21 K, *i.e.* a second inner wall portion 21 K, of the discharge nozzle 6K in parallel fashion as most live insects will descend into the bottom chamber part 4K and the discharge nozzle 6K along inner walls thereof. In an embodiment, the second inner wall portion 21K may be located somewhere halfway the aforementioned converging section "C" of the discharge nozzle 6K as sufficient convergence and compaction of live insects will have occurred at this location for the second injected air flow F2K to be adequate for air suspension/cushioning separated live insects.

As mentioned earlier, the converging section "C" may comprise various profiles of the second inner wall portion 21K and that the substantial parallel engagement between the second injected air flow F2K and the second inner wall portion 21K may occur closer or further away from the second air injection conduit 20K.

To facilitate the substantial parallel engagement between the second injected air flow F2K and the second inner wall portion 21K, an embodiment is provided wherein the discharge nozzle 6K comprises a second inner wall portion 21K which is arranged at a further wall angle β₂K, *i.e.* a second wall angle β₂K, with respect to the longitudinal axis LK of the discharge nozzle 6K. The second injection angle α₂K of the second air injection conduit 20K is then substantially equal/aligned with the second wall angle β₂K. In this embodiment the second inner wall portion 21K is at least locally arranged at the second wall angle β₂K which substantially coincides with the second injection angle α₂K. This alignment of angles α₂K, β₂K allows the second injected air flow F2K to engage the second inner wall portion 21K in substantial parallel fashion for good air suspension/cushioning of the separated live insects when descending into the discharge nozzle 6K along inner walls thereof, e.g. the second inner wall portion 21K. This embodiment may be further clarified by imagining a tangent line T₂K coinciding with the second inner wall portion 21K and wherein the tangent line T₂K is at the second wall angle β₂K. As depicted in Figure 4A, the second inner wall portion 21K may be (slightly) curved without significantly deviating from the tangent line T₂K as depicted. In an embodiment, the second injection angle α₂K of the second air injection conduit 20K is not smaller than the second wall angle β₂K to further ensure that substantial parallel engagement between the second injected air flow F2K and the second inner wall portion 21 K is achieved.

Further, the air injection member 10K may comprise an air inlet 14K for connecting the air injection member 10K to the secondary air source (not shown) and wherein the air inlet 14K is fluidly (gaseous) connected to the main discharge conduit 8K allowing air injection back into the discharge nozzle 6K. In an exemplary embodiment, the air inlet 14K may be fluidly (gaseous) connected to the air chamber 15K, *i.e.* the first air chamber 15K, thereby allowing for the injected air flow F1K, *i.e.* the first injected air flow F1K, in a direction back into the discharge nozzle 6K. In a further exemplary embodiment, the air injection member 10K may comprise a further air inlet (not shown), *i.e.* a second air inlet, which is fluidly (gaseous) connected to the second air chamber 19K, thereby allowing for the second injected air flow F2K in a direction back into the discharge nozzle 6K.

By using a first and second air inlet it is possible to provide the first and second injected air flows F1K, F2K through the first and second air injection conduits 16K, 20K.

From Figure 3A and 4A it is seen that, in an embodiment, the first and second air chambers 15K, 19K may be arranged on opposite sides of the main discharge conduit 8K, providing air in distributed fashion throughout the air injection member 10K of the discharge end 7K. Then in an advantageous embodiment the oppositely arranged first and second air chambers 15K, 19K may be fluidly (gaseous) connected to one another, so that a single air inlet 14K as shown in Figure 2 and 4A may be provided for providing air to both the first and second air chambers 15K, 19K.

By fluidly (gaseous) connecting first and second air chambers 15K, 19K, an embodiment is conceivable wherein the first and second air chambers 15K, 19K form a circumferentially arranged air chamber encircling the main discharge conduit 8K. Such a circumferentially arranged air chamber allows for further equal air distribution throughout the air injection member 10K toward the first and second air injection conduits 16K, 20K.

In line with an opposing arrangement of the first and second air chambers 15K, 19K, and as depicted in Figure 3A and 4A, an embodiment can be provided wherein the first and second air injection conduits 16K, 20K may be arranged on opposite sides of the main discharge conduit 8K. By arranging the first and second air injection conduits 16K, 20K in opposing fashion, it is possible to provide a combination of the first and second injected air flows F1K, F2K into the discharge nozzle 6K that is more symmetrical and evenly distributed there through. Providing improved distribution of injected air back into the discharge nozzle 6K facilities a more uniform air suspension/cushioning of separated live insects, thus further minimizing any unwanted discharge of live insects during an air injection cycle of the air injection member 10K.

Turning to Figure 4B, in this figure the indicated cross section "IV B" (see Figure 4A) of a discharge end 7K of a discharge nozzle 6K is shown. In this embodiment it is clearly depicted that the second air injection conduit 20K extends between and fluidly (e.g. gaseous) connects the second air chamber 19K and the main discharge conduit 8K. It is further shown that the second air injection conduit 20K may be a straight conduit extending between the second air chamber 19K and a further discharge conduit wall portion 22K, *i.e.* a second discharge conduit wall portion 22K, to provide a shortest path from the second air chamber 19K to the main discharge conduit 8K to minimize pressure loss and maximize the intensity of the second injected air flow F2K.

In an embodiment, the second air injection conduit 20K may have a width W₂K *i.e.* a second width W₂K, between 0.2 mm and 1 mm to allow sufficiently strong air volume flowing back into the discharge nozzle 6K for air suspension/cushioning of separate live insects. It is noted that in this embodiment a smaller second width W₂K within this range will generally provide a faster second injected air flow F2K with less air usage compared to having a larger second width W₂K in this range for the second air injection conduit 20K. Choosing smaller values for the second width W₂K will typically lead to less disturbance of the air flow within the discharge nozzle 6K.

When the first and second air injection conduits 16K, 20K are arranged on opposite sides of the main discharge conduit 8K, then this implies that the first and second discharge conduit wall portions 18K, 22K, at which the first and second air injection conduits 16K, 20K terminate, are also oppositely arranged with respect to the main discharge conduit 8K.

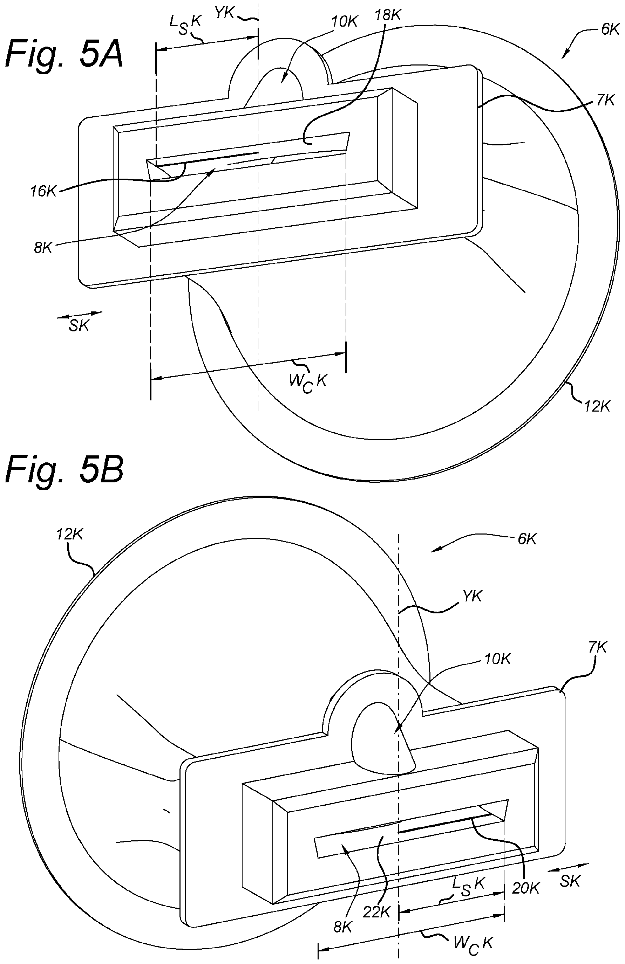

To further elaborate on the indicated cross sections "III A" and "IV A" in Figures 2, 3A, 4A, in Figure 5A a three dimensional view is shown of the main discharge conduit 8K with the first air injection conduit 16K, and Figure 5B shows a three dimensional view of the main discharge conduit 8K from a different angle showing the second air injection conduit 20K. Both Figures 5A and 5B pertain to the same embodiment of the discharge nozzle 6K.

From Figure 5A it is seen that in an embodiment the air injection conduit 16K, i.e. the first air injection conduit 16K, may be a slit shaped conduit, allowing for a widened first injected air flow F1K providing improved air distribution for air suspension/cushioning of live insects within the discharge nozzle 6K. The slit shaped first air injection conduit 16K extends in a lateral direction indicated by "SK" between the first air chamber 15K and the first discharge conduit wall portion 18K of the main discharge conduit 8K. This embodiment ensures that a wide/planar first injected air flow F1K is achieved for improved air suspension/cushioning of separated live insects. In a further embodiment, the slit shaped first air injection conduit 16K may have a width W₁K of about 0.2 mm to 1 mm, thereby allowing for sufficiently strong volumes of air flowing back into the discharge nozzle 6K.

From Figure 5B it is seen that in an embodiment the second air injection conduit 20K may be a slit shaped conduit, allowing for a widened second injected air flow F2K providing improved air distribution for air suspension/cushioning of live insects within the discharge nozzle 6K. As shown, the slit shaped second air injection conduit 20K extends in a sideways/lateral direction indicated by "SK" between the second air chamber 19K and the second discharge conduit wall portion 22K of the main discharge conduit 8K. This embodiment also ensures that a wide/planar second injected air flow F2K is achieved for improved air suspension/cushioning of separated live insects. In a further embodiment, the slit shaped second air injection conduit 20K may have a width W₂K of about 0.2 mm to 1 mm, thereby allowing for sufficient air volume flowing back into the discharge nozzle 6K.

From Figure 5A and 5B it is further seen that the first and second air injection conduits 16K, 20K may be arranged on opposite sides of the main discharge conduit 8K. That is, the first and second discharge conduit wall portions 18K, 22K being arranged on opposite sides of the main discharge conduit 8K. Such an opposing arrangement of slit shaped first and second air injection conduits 16K, 20K allows for a further even distribution of back flowing air into the discharge nozzle 6K for optimal air suspension/cushioning of separated live insects.

In an embodiment, as exemplified in Figure 5A and 5B, the first and second air injection conduits 16K, 20K (e.g. slit shaped conduits 16K, 20) may be sideways/laterally offset or shifted in opposing directions in the indicated sideways/lateral direction "SK" with respect to the discharge nozzle 6K. Sideways/lateral offsetting the first and second air injection conduits 16K, 20K in opposite direction allows for a back flowing air vortex "V" (see Figure 2) to be generated by the first and second injected air flows F1K, F2K, so that an even further improved distribution of air suspension/cushioning of live insects is achieved.

In an advantageous embodiment, the first and second air injection conduits 16K, 20K are arranged to provide a back flowing vortex V exhibiting a rotational direction identical to a rotational direction of the main vortex in the top chamber part 3K which is responsible for centrifugal separation of the live insects from the air streams A. Having identical rotational directions of the main vortex and the back flowing vortex V prevents that rotationally moving live insects descending into the discharge nozzle 6K could potentially stop rotating by an oppositely rotating back flowing vortex V. As a result, live insects could come into prolonged contact with inner walls of the discharge nozzle 6K increasing the chance of clump formation.

In an embodiment, the slit shaped first and second air injection conduits 16K, 20K may each have a length Ls of at most 50% of a width WcK of the main discharge conduit 8K, thereby allowing the first and second injected air flows F1K, F2K to generate a back vortex V within the discharge nozzle 6K through appropriate placement of the slit shaped first and second air injection conduits 16K, 20K. For example, by lateral/sideways offsetting slit shaped first and second air injection conduits 16K, 20K, and limiting the length Ls of each of these conduits 16K, 20K to at most 50% of the width WcK of the main discharge conduit 8K, then a stable and uniform back flowing vortex V can be generated through the first and second injected air flows F1K, F2K for optimal air suspension/cushioning of live insects. Of course, in further embodiments it would be possible that the slit shaped first and second air injection conduits 16K, 20K may each have a length Ls of more than 50% of the width WcK of the main discharge conduit 8K, thereby allowing for further improvements of the first and second injected air flows F1K, F2K if necessary. In even further embodiments the slit shaped first and second air injection conduits 16K, 20K may each have a length Ls between 0 and 100% of the width WcK of the main discharge conduit 8K in case full design freedom of each of the conduits 16K, 20K is required for achieving a specific back flowing air profile into the discharge nozzle 6K.

For example, Figure 5A and 5B show an embodiment wherein the intake end 12K of the discharge nozzle 6K is circular whereas the discharge end 7K is substantially rectangular, i.e. comprising a substantially rectangular main discharge conduit 8K. Both the first and second air injection conduits 16K, 20K are seen to be slit shaped conduits, wherein the first air injection conduit 16K is arranged on a left side of a lateral centreline "YK" of the main discharge conduit 8K whereas the second air injection conduit 20K is arranged on a right side of the centreline "YK". So based on the view provided in Figure 5A, the slit shaped first air injection conduit 16K laterally extends in a top left corner of the substantially rectangular main discharge conduit 8K, and based on the view provided in Figure 5B, the second air injection conduit 20K laterally extends in a bottom right corner of the substantially rectangular main discharge conduit 8K. This embodiment then provides a good back flowing vortex V for air suspension/cushioning of live insects.

Furthermore, since the discharge nozzle 6K changes from a circular geometry at the intake end 12K toward a rectangular geometry at the discharge end 7K, enhances the generation of a back flowing vortex V as the first and second injected air flows F1K, F2K engage and follow a curvature of the first and second inner wall portions 17K, 21K.

As further depicted in figure 5A and 5B, in an embodiment both the slit shaped first and second air injection conduits 16K, 20K may have a length Ls smaller than the width WcK of the main discharge conduit 8K and that an opposing lateral/sideways offset of these conduits 16K, 20K, *i.e.* an offset in opposing directions along the indicated direction SK, is chosen such that both the first and second air injection conduits 16K, 20K do not extend beyond/across the lateral centreline "YK" as depicted. In such a configuration the first and second injected air flows F1K, F2K will not directly collide and a such a smooth and uniform back flowing vortex V can be generated with minimal turbulence in the main discharge conduit 8K.

As mentioned earlier, in an embodiment the discharge nozzle 6K may have a circular intake end 12K and a substantially rectangular discharge end 7K, i.e. with a substantially rectangular main discharge conduit 8K.

This not only facilitates generation of a back flowing vortex V as explained above, but having a substantially rectangular main discharge conduit 8K is also advantageous for reasons related to reliably counting the number of live insects being discharged as explained below.

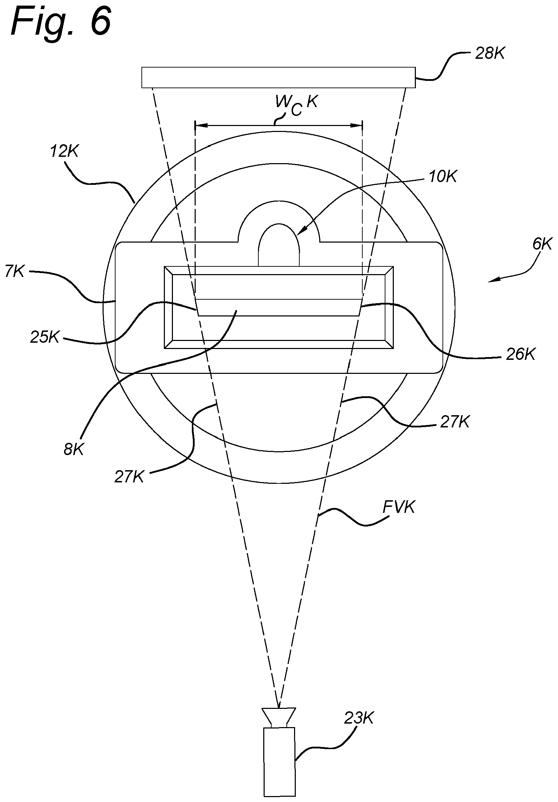

In particular, Figure 6 shows a schematic view in the direction "A" as indicated in Figure 2, wherein an embodiment is depicted comprising a camera based counting system 23K arranged at the discharge end 7K of the discharge nozzle 6K. In this embodiment, the cyclone separation system 1K may further comprise a camera based counting system 23K which is arranged to count the number of live insects being discharged from the main discharge conduit 8K when the cyclone separation system 1K is in operation. Then based on the counted live insects, the air injection member 10K can be activated to momentarily stop the discharge of live insects from the discharge nozzle 6K such that a batch of live insects is collected and can be transferred for further processing.

For example, in Figure 1 and 6 the camera based counting system 23K is depicted and a container 24K is arranged on a transportation system 25K, *e.g.* a conveyor belt, a roller conveyor and the like. Let the cyclone separation system 1K be in operation and live insects are separated and discharged from the discharge nozzle 6K through the main discharge conduit 8K thereof. The camera based counting system 23K is active and counts the number of live insects that pass through its triangular field of view "FVK" . At some point a desired number of live insects has been collected in the container 24K and should be transferred for further processing. To that end the air injection member 10K is activated for a predetermined amount of time to be sufficient for moving the container 24K out of the way and to place another or different container 24K below the discharge nozzle 7K. Therefore, the camera based counting system 23K further facilities accurate control of batches of live insects being discharged based on the actual number of live insects discharged and counted, so that the air injection member 10K can be activated to momentarily suspend/cushion live insects in air within the discharge nozzle 6K to stop the discharge. Once the discharge comes to a stop, the container 24K with collected live insects can be replaced with another or different container, which may or may not be empty, e.g. when holding some food for the live insects to be discharged into the container. Once the other or different container is positioned correctly, the air injection member 10K can be deactivated to resume collection of separated live insects being discharged from the discharge nozzle 6K.

Now, to facilitate accurate and reliable operation of the camera based counting system 23K, in an advantageous embodiment the main nozzle discharge conduit 8K is rectangular such that the live insects discharged there through form a relatively wide but thinner "curtain" or "cloud" of live insects. That is, having a wider and thinner stream of live insects discharged from the discharge nozzle 6K reduces the chance that live insects closer to a camera block the view of live insects behind them. So by ensuring that the field of view FVK extends through a widest side of the rectangular main discharge conduit 8K, facilitates accurate counting of discharged live insects.

In a further advantageous embodiment, the camera based counting system 23K defines a planar triangular field of view FVK and wherein the main discharge conduit 8K comprises a trapezium shaped cross section having two opposing non-parallel sides 25K, 26K each of which is parallel to an edge 27K of the planar triangular field of view FVK. This ensures that the entire trapezium shaped cross section of the main discharge conduit 8K can be monitored by the camera based counting system 23K and that no blind corners of the main discharge conduit 8K exist through which live insects may be discharged undetected.

Of course, in case the main discharge conduit 8K is rectangular, i.e. all sides thereof are perpendicular, then a wider triangular field of view FVK would be needed to avoid blind corners of the main discharge conduit 8K.

In an embodiment, the camera based counting system 23K comprises a light source 28K arranged opposite the main discharge conduit 8K for easier detection through illumination of live insects passing through the field of view FVK. In a further embodiment the light source 28K may be an elongated, line light source 28K, allowing substantially equal light intensity along the cross section of the main discharge conduit 8K. In a further embodiment the camera based counting system 23K may comprise a line scanning camera allowing for the aforementioned planar, triangular field of view FVK.

Referring to Figure 3A, 3B, showing an embodiment of the first air injection conduit 16K in the first discharge conduit wall portion 18K, it is worth noting that the air injection angle α₁K need not be chosen to align with the first wall angle (β₁K) to effectively stop discharge of live insects by injecting air back into the discharge nozzle 6K through the first injection conduit 16K.

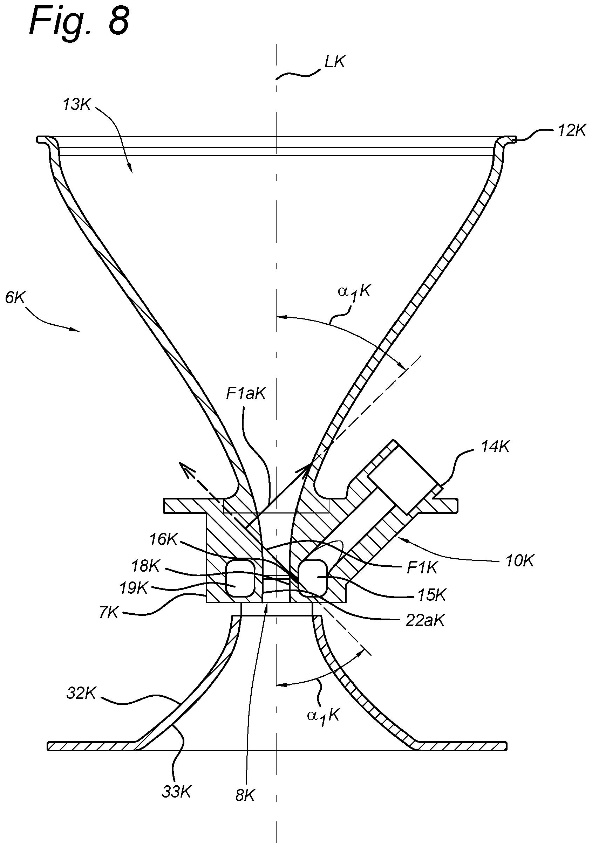

In particular, Figure 8 shows another cross section of a discharge nozzle 6K according to an embodiment of the present invention. In the embodiment shown, a larger air injection angle α₁K may be chosen such that the first injected air flow F1K impinges on an opposing deflection conduit wall portion 22aK of the discharge conduit 8K, wherein the opposing deflection conduit wall portion 22aK is arranged opposite the first discharge conduit wall portion 18K. The deflection conduit wall portion 22aK may be seen as the second discharge conduit wall portion 22K as mentioned earlier. By allowing the first injected air flow F1K to impinge on the opposing conduit wall portion 22aK. *i.e.* the second discharge conduit wall portion 22K, the first injected air flow F1K is deflected from the deflection conduit wall portion 22aK forming a deflected first injected air flow F1aK. In this way a cross-wise air flow is achieved by the air flows F1K, F1aK, thereby allowing for an effective and reliable way of temporarily blocking the discharge of live insects from the main discharge conduit 8K.

In an exemplary embodiment, in orderto achieve such cross-wise flow as depicted in Figure 8, an embodiment is provided wherein the first injection angle α₁K lies between 40° and 60° degrees with respect to the longitudinal axis LK of the discharge nozzle 6K, e.g. wherein the first injection angle α₁K is about 45° degrees. This embodiment allows the first injection angle α₁K to be chosen such that the first injected air flow F1K impinges on the deflection conduit wall portion 22aK producing the deflected first injected air flow F1aK.

It is worth noting that, in further embodiments, larger first injection angles α₁K are also conceivable, e.g. between 60° and 90°, in order to achieve impingement of the first injected air flow F1K on the opposing deflection conduit wall portion 22aK (22K) so that a cross-wise flow F1K, F1aK is obtained for temporarily blocking discharge of live insects from the main discharge conduit 8.

Referring to Figure 4A and 4B, note that from the above embodiment it follows that a larger second injection angle α₂K may likewise be provided to achieve a cross-wise deflected second injected air flow F2K. That is, in an embodiment, the second injection angle α₂K lies between 40° and 60° degrees with respect to the longitudinal axis LK of the discharge nozzle 6K, e.g. wherein the second injection angle α₂K is about 45° degrees. In this embodiment the second injected air flow F2K impinges on the first conduit wall portion 18K in a manner similar to the first injected air flow F1K, thereby achieving a cross-wise flow for temporarily stopping live insects from being discharged from the main discharge conduit 8K.

In further embodiments, larger second injection angles α₂K are conceivable, e.g. between 60° and 90°, in order to achieve impingement of the second injected air flow F2K on the opposing first conduit wall portion 18K, so that a cross-wise flow is obtained for temporarily stopping live insects from being discharged from the main discharge conduit 8K.

Achieving deflected first and second injected air flows F1K, F2K may be advantageous in an embodiment wherein slit shaped first and second air injection conduits 16K, 20K are arranged on opposite sides of the main discharge conduit (8K) and are laterally/sideways offset in opposite direction. This would then result in offset deflected air flows along the main discharge conduit 8K for temporarily stopping discharge of live insects.

In an embodiment mentioned earlier, the slit shaped first and second air injection conduits 16K, 20K may each have a length LsK of at most 50% of a width WcK of the main discharge conduit 8K. Then by offsetting the slit shaped first and second air injection conduits 16K, 20K along the main discharge conduit 8K allows a full air block to be achieved thereof.

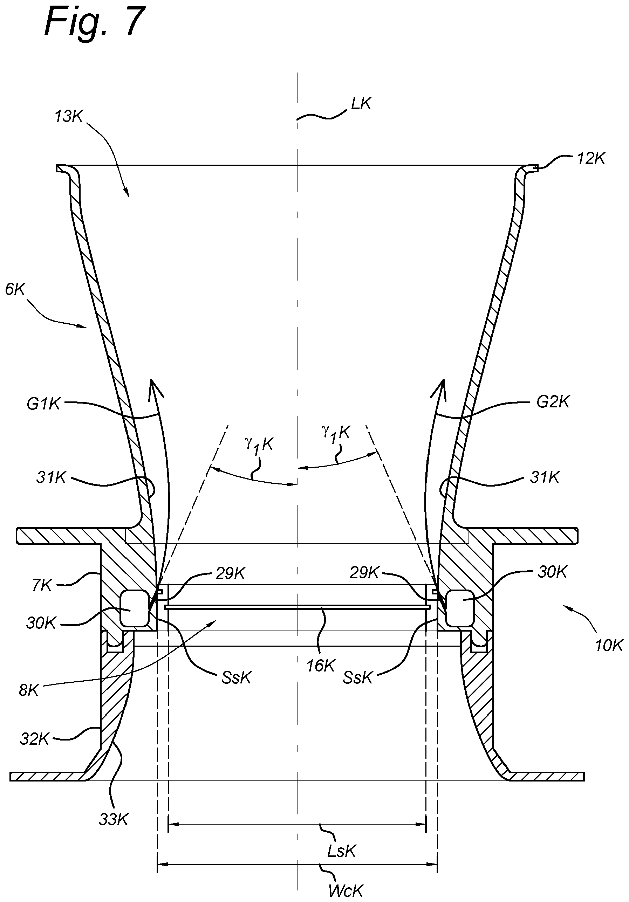

In an advantageous embodiment it is also possible to utilize a single slit shaped air injection conduit, e.g. only using a slit shaped first air injection conduit 16K as shown in Figure 7. This figure depicts another cross section of a discharge nozzle 6 wherein the slit shaped first air injection conduit 16K has a length LsK of more than 50% of a width WcK of the main discharge conduit 8K, e.g. more than 75%, e.g. more than 90%, e.g. more than 95% , *e.g.* 100% of the width WcK. In this embodiment, increasing the length LsK improves the blocking of live insects when air is being injected by the slit shaped first air injection conduit 16K. As shown in Figure 7, in an exemplary embodiment the length LsK of the slit shaped first air injection conduit 16K is at least 90% of the width WcK of the main discharge conduit 8K for providing an air "curtain" to temporarily stop discharge of live insects. Note that this embodiment allows the first injected air flow F1K to extend along the entire main discharge conduit 8K.

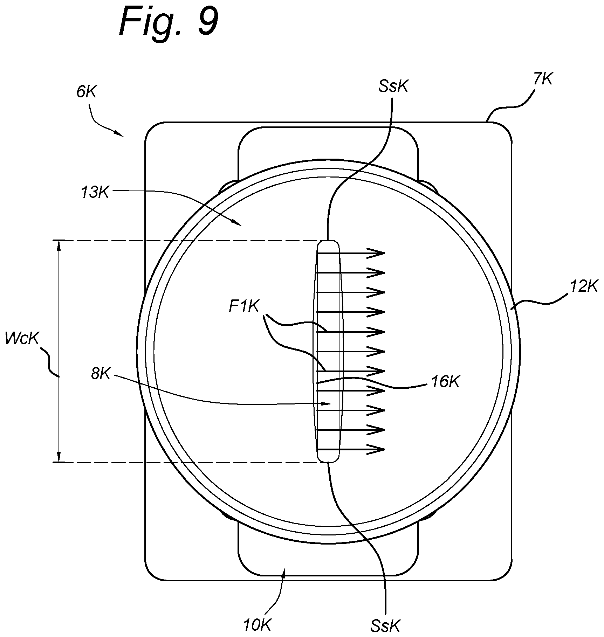

Figure 9 shows a top view of an intake end 12K of the discharge nozzle 8K, wherein the length LsK of the slit shaped first air injection conduit 16K is at least 90%, e.g. 95 %, of the width WcK of the main discharge conduit 8K so that the first injected air flow F1K substantially extends along the entire length of the main discharge conduit 8K.

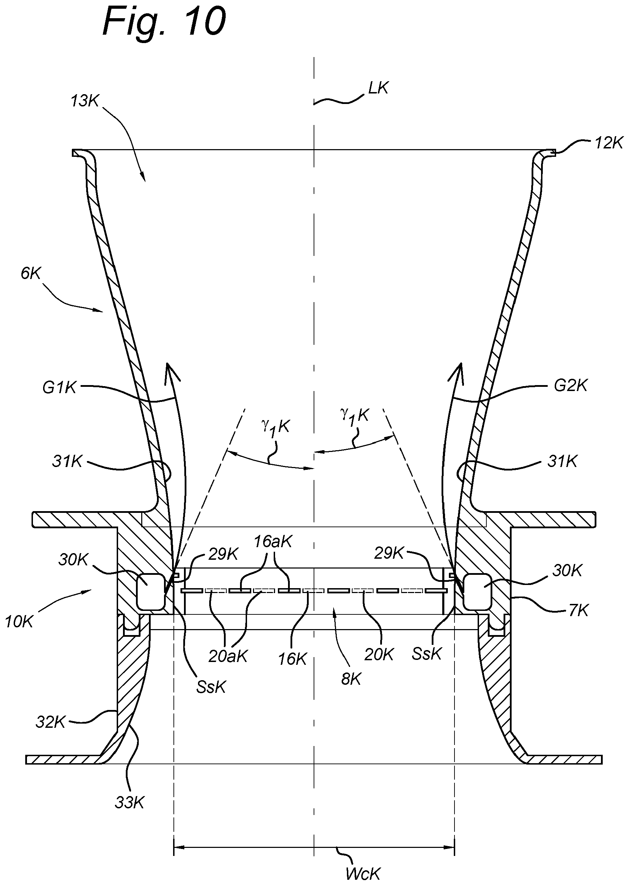

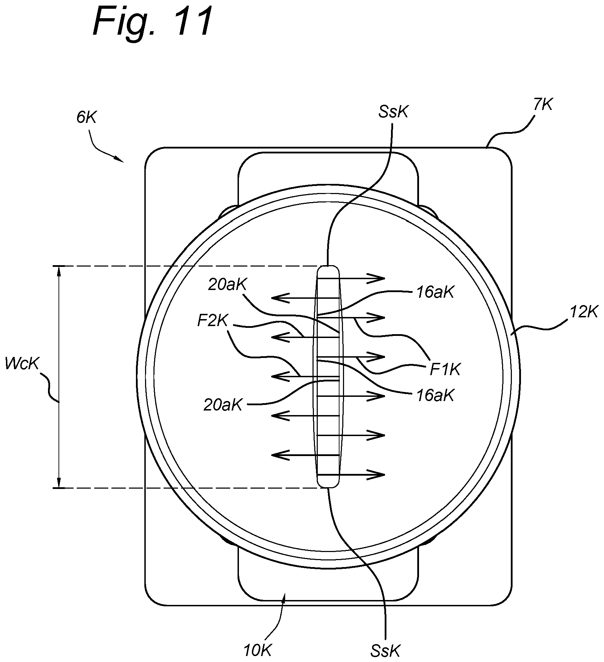

Instead of using a single first air injection conduit 16K along the main discharge conduit 8K as exemplified in Figure 7 and 9, it is also possible to utilize an alternating arrangement of a plurality of opposing first and second air injection conduits 16K, 20K as shown in Figures 10 and 11, wherein Figure 10 shows another cross section of a discharge nozzle 6K and wherein Figure 11 shows another top view of an intake end 12K of a discharge nozzle 6K according to an embodiment of the present invention.

In the depicted embodiments, the first air injection conduit 16K may comprise a plurality of first conduit sections 16aK and wherein the second air injection conduit 20K may comprise a plurality of second conduit sections 20aK, wherein the plurality of the first and second conduit sections 16aK, 20aK are laterally/sideways offset in alternating manner along a width WcK of the main discharge conduit 8K. In this embodiment the first and second conduit sections 16aK, 20aK may provide an alternating arrangement of opposing first and second injected air flows F1K and F2K as shown in Figure 11 for temporally blocking discharge of live insects.

Referring again to Figure 7 and Figure 10, in the depicted embodiments the air injection member 10K of the discharge end 7K may comprises two opposing auxiliary air chambers 30K and two opposing auxiliary injection conduits 29K each of which fluidly connects one of the two auxiliary air chambers 30K with the main discharge conduit 8K of the discharge end 7K. Each auxiliary injection conduit 29K is then arranged to provide an auxiliary injected air flow G1K, G2K in a direction back into the discharge nozzle 6K. In this embodiment the main discharge conduit 8K is considered to have a substantially rectangular shape, e.g. a substantially rectangular cross section as depicted in Figure 9 and 11, wherein the two opposing auxiliary air chambers 30K and the two opposing auxiliary injection conduits 29K are arranged along (opposing) shortest sides SsK of the substantially rectangular shaped main discharge conduit 8K. In this embodiment, having two auxiliary injected air flows G1K, G2K at the shortest sides SsK of the main discharge conduit 8K prevents accumulation of live insects at inner wall portions of the shortest sides SsK. In particular, since the first and/or second injected air flows F1K, F2K along the longest sides of the main discharge conduit 8K as depicted may not be able to provide sufficient air flow along these inner wall portions, the two auxiliary injected air flows G1K, G2K may improve removal of live insects along the shortest sides SsK in addition to the first and/or second injected air flows F1K, F2K.

To further prevent accumulation of live insects, an advantageous embodiment is provided wherein each of the two auxiliary air injection conduits 29K is arranged at an auxiliary injection angle γ₁K between 10° and 50° degrees with respect to the longitudinal axis LK of the discharge nozzle 6K. In this embodiment the auxiliary injection angle γ₁K of the two auxiliary air injection conduits 29K may be chosen to prevent separation of the two auxiliary injected air flows G1K, G2K from an inner surface 31K of the shortest sides SsK, which inner surface 31K extends from the shortest sides SsK into the funnel shaped passage 13K. Therefore, when the two auxiliary injected air flows G1K,G2K remain attached to the inner surface 31K improves air flow there along and as such prevent accumulation of live insects at the inner surface SsK of the shortest sides SsK of the main discharge conduit 8K. In an exemplary embodiment the auxiliary injection angle γ₁K is about 45° degrees, or even 35° degrees, to prevent separation of the two auxiliary injected air flows G1K, G2K from the inners surface 31K of the shortest sides SsK of the main discharge conduit 8K.

Referring shortly to Figure 1, is was mentioned earlier that the camera based counting system 23K may be provided and a container 24K may be arranged underneath the discharge nozzle 6K on a transportation system 25K for collecting a batch of live insects. When the cyclone separation system 1K is in operation, and live insects are separated and discharged from the discharge nozzle 6K through the main discharge conduit 8K, the camera based counting system 23K is able to count the number of live insects passing through the field of view "FVK" of the camera based counting system 23K. Once a desired number of live insects has been collected in the container 24K, discharge of the live insects can be temporarily stopped by injecting air back into the discharge nozzle 6K through the first and/or second air injection conduits 16K, 20K. During that time, a new container may be placed underneath the discharge nozzle 6K.

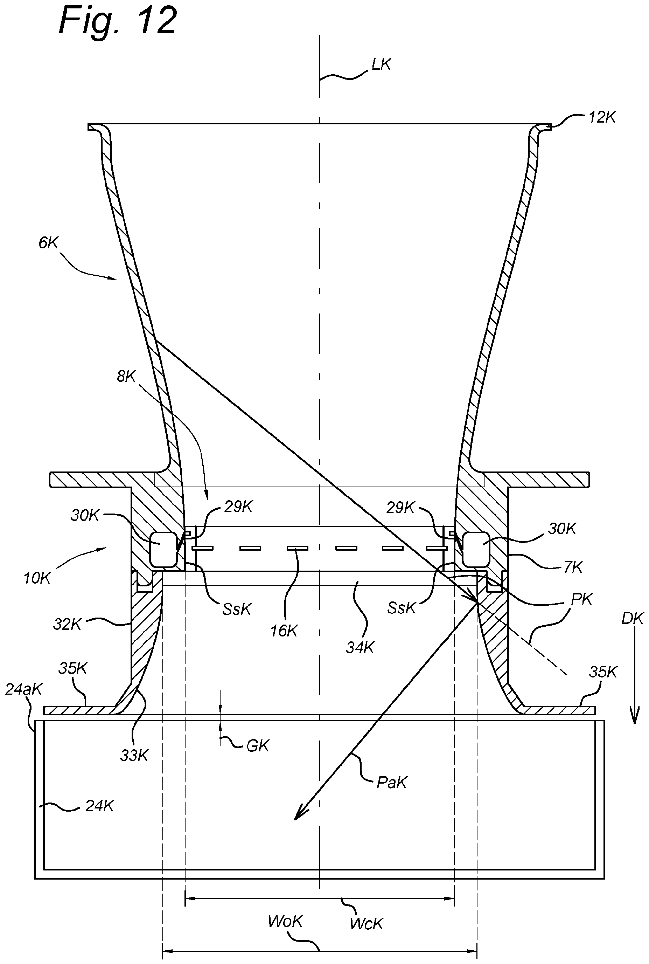

However, when live insects are discharged into the container 24K, a number of the live insects may not be discharged parallel to the longitudinal axis LK of the discharge nozzle 6K. That is, a particular number of live insects could potentially be discharged from the main discharge conduit 8K in a diagonal manner as shown in Figure 12, which shows another embodiment of a discharge nozzle 6K and a container 24K arranged thereunder. As depicted, live insects could potentially follow a diagonal discharge path/trajectory PK through the main discharge conduit 8K and as a result the live insects in question would not be discharged in the container 24K.

According to the present invention, to prevent live insects from missing the container 24K, an embodiment is provided wherein the discharge nozzle 6K further comprises a discharge guiding member 32K mounted to/underneath the discharge end 7K of the discharge nozzle 6K. The discharge guiding member 32K comprises an expanding guiding channel 33K fluidly coupled to the main discharge conduit 8K for receiving live insects when the cyclone separation system 1K is in operation. In this embodiment the guiding channel 33K expands in the downstream direction DK as depicted. This embodiment allows live insects to follow the discharge path PK out of the main discharge conduit 8K but to be deflected by the guiding channel 33K and follow a deflected discharge path PaK into the container 24K. The discharge guiding member 32K thus ensures that live insects are discharged into the container 24K by deflecting live insects from the main discharge conduit 8K into a deflected trajectory PaK toward the container 24K.

From Figure 12 it is further seen that in a preferred embodiment the discharge guiding member 32K and guiding channel 33K thereof may engage a circumferential rim portion 24aK of the container 24K, thereby minimizing a vertical gap GK between the discharge guiding member 32K and the container 24K to ensure all live insects are caught by the container 24K.

In an embodiment, the discharge guiding member 32K may further comprise a lower circumferential rim portion 35K, e.g. a circumferential flange portion, that engages the circumferential rim portion 24aK of the container 24K. The lower circumferential rim portion 35K can be used, for example, to cover a part of the container 24K when the guiding channel 33K is less wide than the container 24K, i.e. less wide that an upper opening of the container 24K.

As further depicted in Figure 12, the discharge nozzle 6K comprises a laterally extending elongated opening 34K arranged between the discharge guiding member 32K and the discharge end 7K, and wherein the laterally extending elongated opening 34K extends parallel along the main discharge conduit 8K. The laterally extending elongated opening 34K has an opening width WoK equal to or larger than a width WcK of the main discharge conduit 8K. The laterally extending elongated opening 34K allows counting of live insects exiting the main discharge conduit 8K. As the opening width WoK is at least equal to the width WcK of the main discharge conduit 8K ensures that all live insects existing the main discharge conduit 8K can be observed.

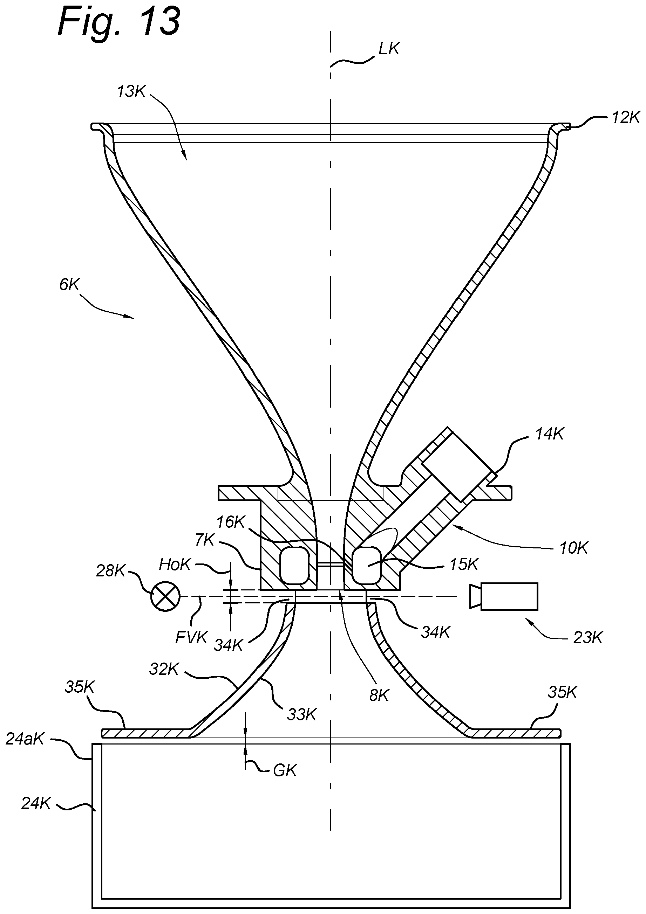

Figure 13 shows another schematic view of a camera based counting system 23K arranged at a discharge end 7K of a discharge nozzle 6K according to an embodiment of the present invention. As depicted, the camera based counting system 23K may comprise a light source 28K arranged on an opposing side of the laterally extending elongated opening 34K through which the field of view FVK is able to extend between the camera based counting system 23K and the light source 28K. This enables accurate counting of live insects exiting the main discharge conduit 8K without any interference. It is worth noting that in an advantageous embodiment the opening height HoK of the laterally extending elongated opening 34K may be small so as to prevent any live insect escaping through the laterally extending elongated opening 34K.

With reference to Figures 1 and 2K, in a further aspect the present invention relates to a method of separating live insects from an air stream AK, and in particular to a method of providing batches of live insects, wherein the method comprises the steps of a) providing a cyclone separation system 1K according to the invention outlined above, and b) connecting each of the one or more intake channels 5K to a primary air source providing an air stream AK comprising live insects and connecting the air injection member 10K to a secondary air source.

Then, assuming the cyclone system 1K is in operation, the method continues with the step of c) collecting separated live insects being discharged from the discharge nozzle 6K. When a prescribed number of live insects have been collected, then the subsequent step of the method comprises the step of d) injecting air back into the discharge nozzle 6K with the air injection member 10K for a predetermined time period to temporarily stop discharge of live insects from the discharge nozzle 6K. In this step the injection member 10K is temporarily deployed to cease discharge of live insects by air suspension/cushioning through the injected air flow F1K, *i.e.* the first injected air flow F1K, or the first and a further injected air flow F2K, i.e. the second injected air flow F2K. Then during the predetermined time period when air injection is active, the method continues with the step of e) transferring the collected live insects away from the discharge nozzle 6K, which step may be associated with exchanging a loaded container 24K for an empty one.

In an embodiment, when the method step e) has been completed, then the method may further comprise the step of f) repeating the steps c) to e), *i.e.* to c) collect separated live insects and when a desired number of live insects have been collected, to d) inject air back into the discharge nozzle 6K for a predetermined time period, and during this predetermined time period, to e) transfer the collected live insects away from the discharge nozzle 6K.

The present invention has been described above with reference to a number of exemplary embodiments as shown in the drawings. Modifications and alternative implementations of some parts or elements are possible, and are included in the scope of protection as defined in the appended claims.

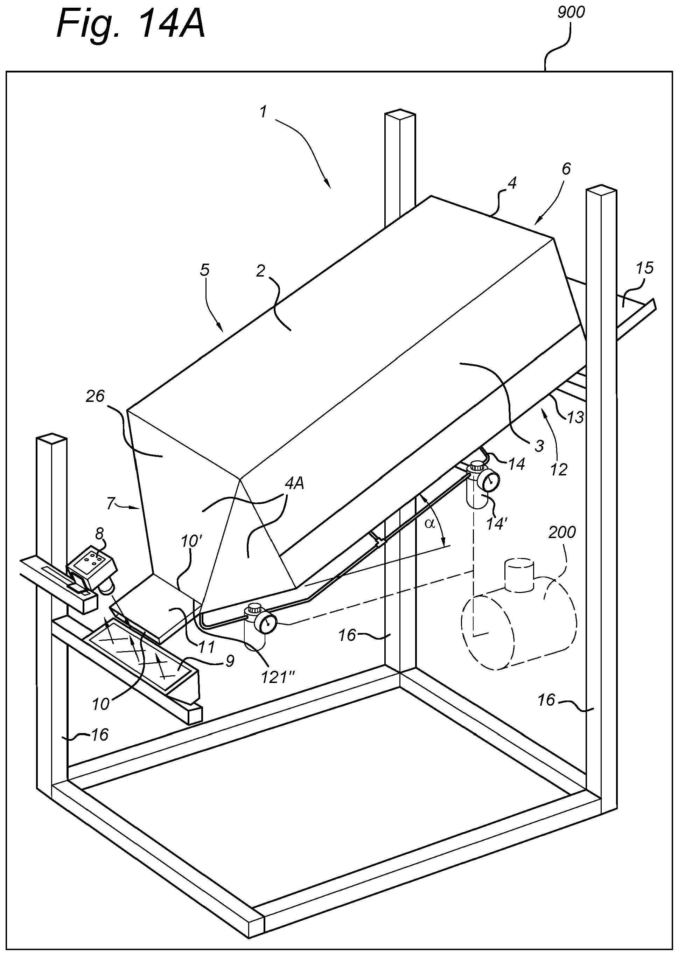

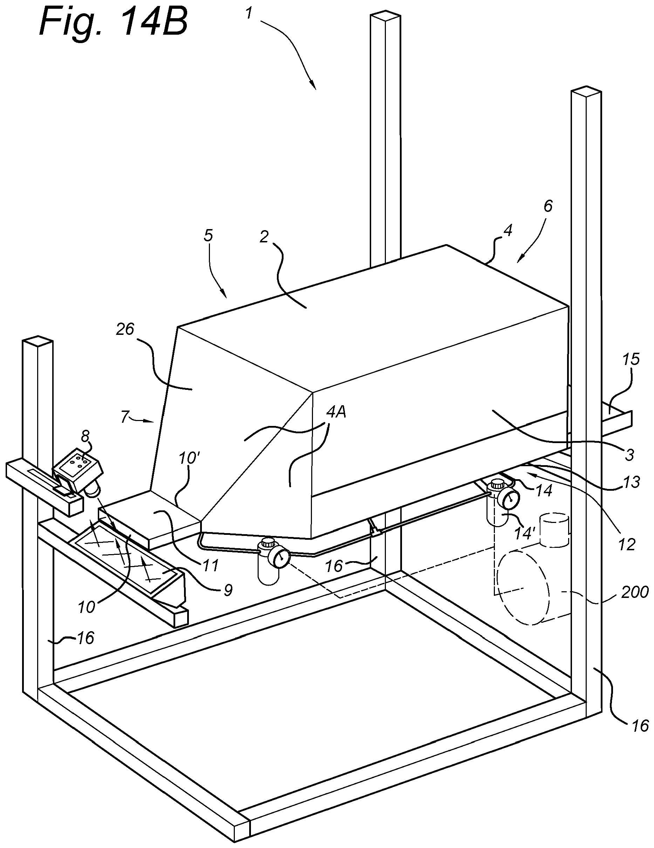

Referring to Figure 14, an overview of an embodiment of the invention is provided, showing a live insects transport device 1. The live insects transport device is optionally tilted relative to the horizontal over an angle α (alpha). Further, an insect discharge member 11 is indicated, provided with a camera 8 and a lamp 9 at the proximal end 10 of the live insect discharge member 11, which is coupled at its distal end 10' to the opening in the side wall 7 of casing 5, at the proximal end 26 of the live insect transport device 1. The camera 8 is a high-speed imager able to detect, image and store images at the speed required for counting and dosing larvae exiting the live insect transport device through the opening of the live insect discharge member located at proximal end 10. Other measurements like determination of lipid content by application of near infra red spectroscopy, could also be performed, for example. The live insects transport device is coupled to a frame 16, amongst others for the purpose of tilting the transport device over said angle α (alpha). Positioning the transport device 1 over said angle prevents larvae from contaminating the lamp 9, positioned in the proximity of the opening of the live insect discharge member 11. The live insects transport device comprises a gas guiding unit 12 comprising upright side walls 13. The transport device further comprises a casing 5 covering, for example a thermally insulated casing 5, the gas guiding unit and the feeder arrangement (not shown), the casing comprising a top wall 2, side walls 3, 4, 4A, 7. Optionally, the side walls and the top wall are provided with a layer of thermally insulating material, such that the casing is thermally insulating the interior of the insects transport device defined by the side walls and top wall of the casing and by the gas guiding member(s). At the distal end 6 of the live insects transport device 1, the distal end 15 of the gas guiding unit 12 is located. Here, a first gas discharge member (not shown) is located, being configured to connect to a source of gas 200. The source of gas comprises a pump or a compressor 14', and the gas is provided to the live insects transport device via tubing or pipes 14, connecting the source of gas to gas discharge members. In an embodiment, side wall 4 is an openable door for providing access to the interior of the insect transport device, from the exterior side. For example, loading the insect transport device 1 with one or more reservoirs 128 is through the opened door 4. Door 4 is provided with a grip 4' and a pivot 4".

Now referring to Figure 15, a drawing is displayed providing an overview of a live insects transport device 1 of the invention comprising a thermally insulated casing 5 and a gas guiding unit 12 that provides a smooth longitudinal path for a laminar flow of gas, and further displays the distal end 15 of the gas guiding unit which receives the gas discharge members 20, 20' through an opening 17 in the casing 5. The gas discharge members 20, 20' are coupled to a source of gas (not shown) with tubing 19 and 19', said tubing coupled to the gas discharge members with couplers 18, 18'. The live insects transport device is further provided with a live insects discharge member 11. The side wall 4 of the casing 5 is an openable door 4 provided with a grip 4' and a pivot 4", for providing access to the interior of the insects transport device, for example for delivery of a reservoir or for removal of an empty reservoir after operation of the insects transport device. The top wall and side wall of the casing 5 are for example thermally insulated walls, provided with a layer of thermally insulating material, such that the volume defined by the casing and the gas guiding unit(s) inside the insects transport device is thermally insulated.

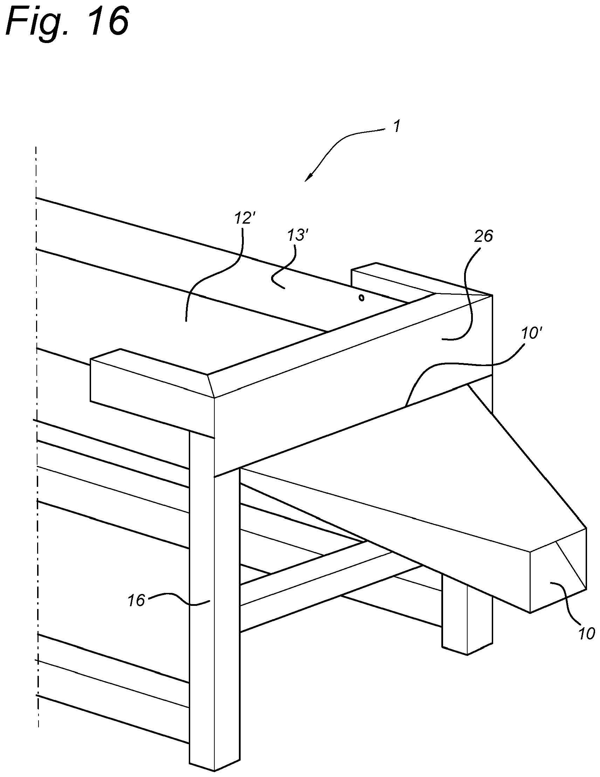

Now referring to Figure 16, a drawing is displayed providing a detailed side view of an insects transport device 1 where the proximal end 26 of the gas guiding unit 12' ends and where the insect discharge member (See also 11 in Figure 15) is located and coupled to said proximal end with the distal end portion 10' of the live insects discharge member. The live insects discharge member has a funnel-like shape, configured to provide a narrowed stream of flowing live insects in the flow of gas exiting the insects transport device. Narrowing the stream of live insects provides the benefit of a smaller cross section of the flow of gas comprising the live insects, in support of counting, sorting and/or dosing the insects. The gas guiding member comprises upright side walls 13'. The live insect receiving zone is provided by the smooth top surface of the gas guiding member 12'.

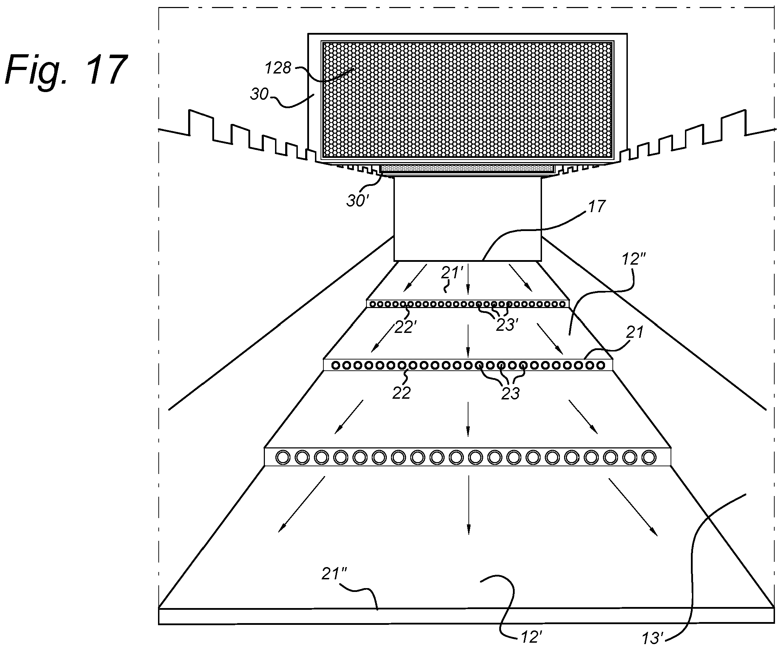

Now referring to Figure 17, a drawing is displayed providing an inside view of an insects transport device. Shown are longitudinal gas transport members 12', 12" which are connected imbricatedly at positions 21, 22 and 21', 22'. Where two consecutive gas transport members are coupled imbricatedly, a gas discharge member (not shown; See 20, 20' in Figure 15 and 114', 114", 114‴ in Figure 18) is positioned at the location where said gas transport members overlap, said gas discharge member provided with openings 23, 23' for discharging gas. In this embodiment, the live insects receiving portion is provided by the smooth top surface of four imbricatedly coupled gas guiding units, two of which are indicated with 12' and 12". The transport device has straight upright walls 13'. The laminar flow of gas is in the direction of the arrows, flowing to the proximal end 21" of the proximal gas guiding member 12'. The feeder arrangement (see 127 in Figure 19) here received a frame 30, 30', encompassing a reservoir 128 for releasing live insects above the live insects receiving portion provided by the smooth top surface of the gas guiding unit.

Now referring to Figure 18, a drawing is displayed providing an overview of another embodiment, showing an insects transport device 100 comprising a live insects receiving portion that is built up by a gas guiding unit 112 comprising side walls 113 tilted at an obtuse angle relative to the top surface of the gas guiding members. The insects transport device of the embodiment comprises a casing 105, said casing comprising thermally insulated side walls 103, 104 and a top side 102, the top side made at least in part from a transparent material 125 such as a plate made of glass, a transparent polymer or polymer blend, *etc.* The insects transport device 100 is provided with a live insects discharge member 111, coupled to the transport device at its distal end 110' at an opening 107 located at the proximal end 126 of the transport device, the live insects discharge member further comprising a proximal end where the laminar flow of gas comprising live insects exits the discharge member. The insects transport device is provided on a frame 106, 116. Gas discharge members 114', 114" and 114‴ are coupled to a gas source via tubing 114, the gas source comprising a compressor unit 124 comprising a pressure control unit 140. Gas discharge members 114', 114" and 114‴ are configured to provide a flow of gas for reinforcing the laminar flow of gas discharged into the insects transport member at the distal end of the gas guiding unit.

Now referring to Figure 19, a drawing is displayed providing a view on part of a live insects receiving portion of an insects transport device 100, the live insects receiving portion being built up by a gas guiding unit 112' comprising side walls 113' and 113" tilted at an obtuse angle (β (beta)) relative to the top surface of the gas guiding members. Further displayed are the proximal end 121" of the live insects guiding unit 112' and the further gas discharge members 131 and 131' located at the top side of the side walls, and the feeder arrangement 127 located above the live insects receiving portion of the top surface of the gas guiding unit. A first laminar flow of gas, such as a laminar flow of air, is provided in the direction of the arrows c towards the direction of the location of the proximal end 121" of the live insects guiding unit 112'. A further laminar flow of gas, yet at a lower pressure and/or at a lower velocity in m³/sec, than the pressure and/or velocity of the gas in the first laminarflow, is provided in the direction of the arrows a and b, provided by the gas discharge members 131' and 131, respectively, wherein gas is discharged through openings 129' and 129, respectively. The feeder arrangement 127 received frames, encompassing a reservoir 128, 128' for releasing live insects above the live insects receiving portion provided by the smooth top surface of the gas guiding unit.

Now referring to Figure 20, a drawing is displayed providing a view of an insects transport device 100 along the longitudinal gas guiding units in the direction towards the first gas discharge member located at opening 117 in the side wall 4, 106 of the transport device 100. Consecutive gas guiding units are connected imbricatedly and at positions where the gas guiding units overlap imbricatedly further gas discharge members are located for reinforcing the first laminar flow of gas. The live insects receiving portion is shown and is built up by a gas guiding unit 112 comprising side walls 113' and 113", e.g. flat side walls 113', 113", tilted at an obtuse angle relative to the top surface of the gas guiding members. Further displayed are the distal end of the live insects guiding unit and the further gas discharge members 131' and 131 located at the top side of the side walls 113" and 131', respectively. The gas discharge members located at positions where consecutive gas guiding members imbricatedly overlap, i.e. positions 121', 122' (i.e. overlap between the proximal end 121' of a first gas guiding member and the distal end 122' of a consecutive gas guiding member) and 121, 122 (i.e. overlap between the proximal end 121 of the second gas guiding member and the distal end 122 of a consecutive third gas guiding member), are provided with openings 123', 123 for providing the first laminar flow of gas in the direction of the arrows c. Further gas discharge members 131' and 131 are provided with openings 129' and 129, for releasing gas such that a laminar flow of gas over the surface of tilted side walls 113" and 113' is provided in the direction of the arrows, perpendicular to the direction of the first laminar flow of gas. Gas discharge members are coupled to a source of gas such as compressed air or a driver for driving air through the gas discharge members such as a pump or a fan, via tubing or pipes 114, the source of gas optionally comprising a control unit 124 for example for controlling the gas pressure at entrance of the live insect transport device and/or for controlling the velocity of the gas provided for the building up of the first and further laminar flows of gas.

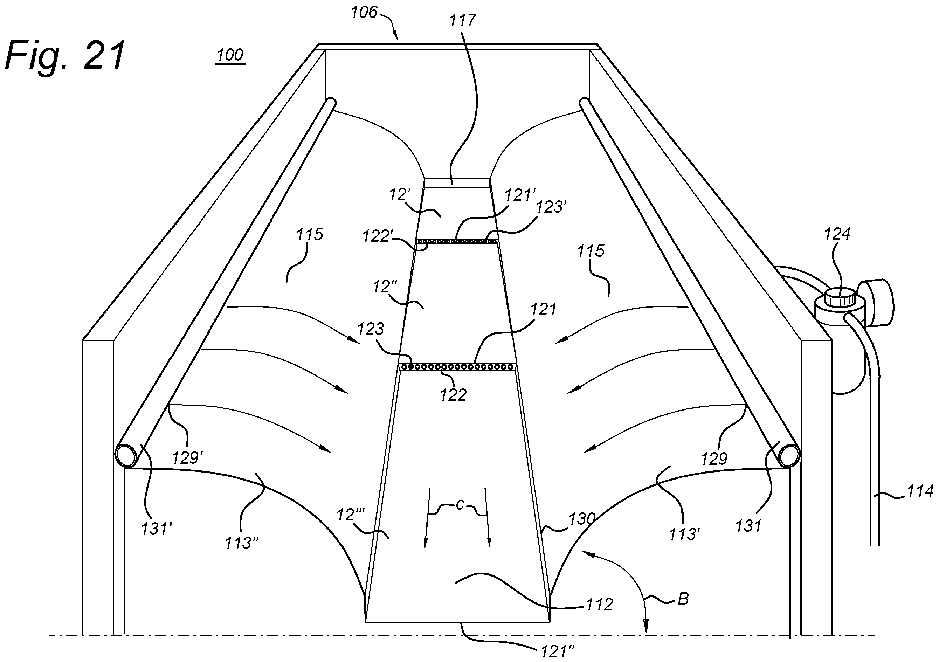

Figure 21 shows an alternative embodiment of the embodiment shown in Figure 20 of an insect transport device 100, wherein the live insects receiving portion further comprises convex side walls 113', 113", i.e. two opposing convex side walls 113', 113", located along longitudinal sides of the at least one longitudinal gas guiding member 12', 12", 12‴, e.g. three longitudinal gas guiding members 12', 12", 12‴, wherein each convex side wall 113', 113" has a top side and a bottom side, and a smooth convex surface 115 arranged and extending there between, and wherein the bottom side is connected to a longitudinal side of the at least one longitudinal gas guiding member 12', 12", 12‴. As further depicted, the top side of each convex side wall 113', 113" is provided with a second gas discharge member 131, 131' comprising a connector configured to connect the second gas discharge member 131, 131' to a source of gas for providing a second laminar flow of gas over the surface 115 of the convex side wall 113', 113" from the top side thereof to the at least one gas guiding member 12', 12", 12‴ during operation of the insect transport device.

In contrast to the embodiment shown in Figure 20, in the embodiment of Figure 21 each side wall 113', 113" is a convex side wall 113, 113" having a top side provided with a second gas discharge member 131, 131' comprising openings 129, 129' for discharging a gas, e.g. air, such that the second laminar flow of gas follows the convex surface 115 toward the at least one longitudinal gas guiding member 12', 12", 12‴.

The convex side walls 113', 113" exhibit the advantageous effect in that when gas such as air flows over the convex side walls 113', 113" toward the top surface of the at least one gas guiding member 12', 12", 12‴, the speed of gas is maintained to a higher degree compared to gas flowing over flat side walls 113', 113" as shown in the embodiment of Figure 20.

For example, when a gas such as air is discharged from the second gas discharge members 131, 131' at a speed of 4 m/sec over flat side walls 113', 113" as depicted in Figure 20, then the air may approach the top surface of the at least one gas guiding member 12', 12", 12‴ at a speed of about 2 m/s. On the other hand, for convex side walls 113', 113" as shown in Figure 21, in order to reach 2 m/s air speed at the top surface of the at least one gas guiding member 12', 12", 12‴, then air may be discharged from the second gas discharge members 131, 131' at a lower speed of e.g. 3 m/s.

In a further example, in case air is discharged from the second gas discharge members 131, 131' at a speed of about 1,2 m/sec, then the air may approach the top surface of the gas guiding members at a speed of about 0,4 m/sec, which is sufficient to maintain suspension of live insects in the first laminar flow of gas, e.g. air, over the top surface of the at least one gas guiding member 12', 12", 12‴.

Therefore, gas flowing over the convex side walls 113', 113" maintains its speed to a much higher degree and a such less gas needs to be discharged by the second gas discharge members 131, 131' for facilitating laminar flow over the top surface of the at least one gas guiding member 12', 12", 12‴ for transport of the live insects.

As the convex side walls 113', 113" allow for lower speeds of air being discharged from the second gas discharge members 131, 131' with minimal loss of momentum, the discharged air has less impact on e.g. environmental conditions (e.g. temperature, humidity) surrounding the reservoirs comprising the live insects. For example, when a thermally insulated casing 5 is provided covering the gas guiding unit 112 and the feeder arrangement as mentioned above, then the convex side walls 113', 113" allow air to be discharged toward the top surface of the at least one gas guiding member 12', 12", 12‴ with reduced impact on environmental conditions on the inner side of the casing 5.

It is further noted that when a gas such as air flows over the convex side walls 113', 113", then the gas tends to closely follow and "stick" to the convex side walls 113', 113" in substantially laminar fashion so that turbulence is kept to a minimum. As a result, laminar flow over the convex side walls 113', 113" reduces the amount of conditioned air being disturbed or pulled away from the at least one reservoir 128, 128' (see Figure 19) and as such the laminar flow over the convex side walls 113', 113" reduces the amount of conditioned air being disturbed or pulled away from insect eggs contained in the at least one reservoir 128, 128'.

In an embodiment, the convex side walls 113', 113" engage the top surface of the at least one gas guiding member 12', 12", 12‴ at an angle (β) between 45 and 60°, such that (laminar) air flowing over the convex side walls 113', 113" causes minimum disturbance of conditioned air around insect eggs contained in the at least one reservoir 128, 128'.

For example, relative humidity of air at 1 bar around the insect eggs or around live insects such as mites may be 80-85% at a temperature of 28°C to 35°C +/- 0.5°C. The second gas discharge members 131, 131' may then discharge a gas, e.g. air, at 1 bar at a temperature of 20°C to 30°C and with relative humidity of 40% - 55%, e.g. 45%. As the discharged air flows in substantially laminar fashion over the convex side walls 113', 113" in a temperature controlled manner, condensation is prevented. Condensation of water vapor inside the casing 5 at any surface of the interior of the insects transport device is further prevented due to the provision of thermally insulated side walls and top wall of the casing. The inventors established that during operation of the insects transport device provided with air feed channel 5A, part of humid 'climate' air fed to the device by feed channel 5A, stays in the cabinet and part of the humid climate air is taken up by the laminar air flow. The volume of the humid climate air is about 20%-40% of the volume of the air building up the laminar air flow and therewith the climate air having a higher humidity than the 'transport' air in the laminar air flow, is sufficiently diluted in the less humid transport air, such that condensation of water vapor is prevented, for example inside the insects transport device and also when the transport air comprising a fraction of the climate air cools down to e.g. ambient temperature of 18°C - 23°C upon exiting the insects transport device, and entering tubing, *etc.*

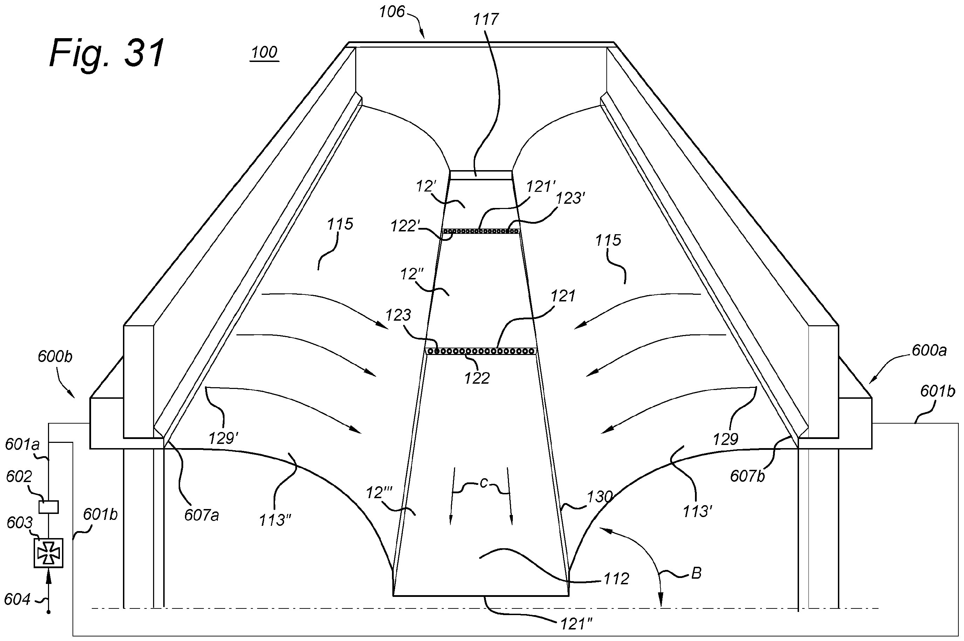

Figure 31 shows an alternative embodiment of the embodiment shown in Figure 21 of an insect transport device 100, wherein the further gas discharge members 131 and 131' located at the top side of the side walls in the embodiment of Figure 20 are now replaced by gas discharge members 600a and 600b, comprising elongated slits 607a and 607b respectively, for discharging gas, e.g. temperature and absolute humidity controlled air, in directions 129' over the convex surface of convex side walls 113', 113". Gas discharge members 600a and 600b are connected to tubing or pipes 601a and 601b, respectively, jointly connected to driver 603 such as a fan 603, which driver 603 drives ambient air through tubing or pipes 601a and 601b towards slits 607a and 607b. The air driven by fan 603 is temperature controlled air and absolute humidity or relative humidity controlled air. Temperature and humidity is controlled with sensor602. The air temperature and air humidity is kept within temperature boundaries and within humidity boundaries suitable for keeping insect alive which are transported through the insect transport device 100 and cyclone separation system 1K.

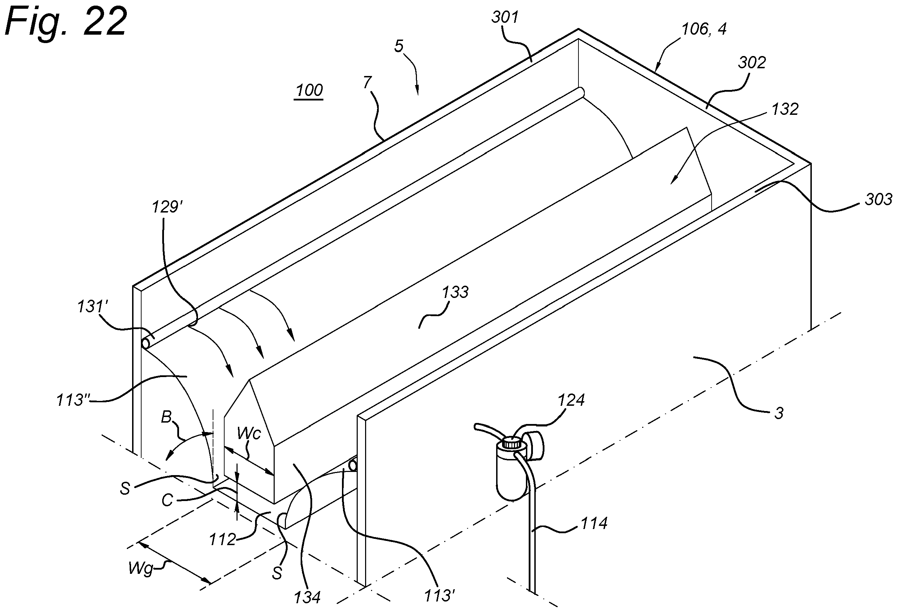

Figure 22 depicts an insect transport device 100 comprising an elongated cover member 132 arranged over and along a gas guiding unit 112. Further, thermally insulating material 301-303 in the side walls of casing 5 are provided for aiding in avoiding condensation of water inside the insects transport device during operation, when temperature drops in the air surrounding the insects transport device may occur.

In the embodiment shown, the insects transport device 100 may be considered to be the same as the one shown in Figure 21 but wherein a cover member 132 is provided that extends above and along the gas guiding unit 112 at a clearance distance "C", thus wherein the cover member 132 extends along and above the at least one gas guiding members 12', 12", 12‴ at a clearance distance "C" with respect thereto. The clearance distance "C" is sufficiently large to allow the first laminar flow of air with live insects, e.g. larvae or live mites, to flow freely over the top surface of each of the at least one gas guiding member 12', 12", 12'" extending underneath the cover member 132.

The cover member 132 prevents that the first laminar flow over the gas guiding unit 112, *i.e.* the at least one gas guiding member 12', 12", 12‴, drags too much conditioned air toward the exit of the insects transport device 100 at a proximal end thereof. In case too much air is being dragged along with the first laminar flow, then this would produce too much turbulence at the exit because of the limited flow capacity there through causing air being lifted upward at the proximal end of the live insect larvae transport device 100.

Therefore, the cover member 132 maintains homogenous distribution of conditioned air around the insect eggs or live mites in the at least one reservoir 128, 128', 128a, 128a' by minimizing the amount of conditioned air being dragged away and/or downward therefrom along with the first laminar flow over the gas guiding unit 112.

In an embodiment, the cover member 132 has a height such that it extends and remains underneath the at least one reservoir 128, 128', 128a, 128a' so that conditioned air around the insect eggs or around the mites is prevented from being dragged with the first laminar flow over the gas guiding unit 112.

In another embodiment, the cover member 132 may further comprise a sloped roof 133 to prevent that live insects collect on the cover member 132 when dropping from the at least one reservoir 128, 128', 128a, 128a' onto the cover member 132, thereby ensuring that the live insects reach the first laminar flow of gas over the gas guiding unit 112.

In a further embodiment, the cover member 132 comprises a plurality of cover side walls 134, e.g. oppositely arranged cover side walls 134, wherein each cover side wall 134 extends in upward and longitudinal/lengthwise direction along one of the convex side walls 113', 113" to further reduce any suction or dragging of conditioned air by the first laminar air flowing over the gas guiding unit 112. Note that lowest edges of each cover side wall 134 are arranged above the gas guiding member 112 at the aforementioned clearance distance C. In a further embodiment, the cover member 132 comprises a bottom side (not visible in Figure 22) which may be an open or a closed bottom side. In case the bottom side is closed, then the bottom side extends along and above the gas guiding unit 112 at the aforementioned clearance distance C.

In an exemplary embodiment, the cover member 132 has a width w_{c} which may be substantially the same as a width W_{g} of the gas guiding unit 112. Since the cover member 132 is arranged above the gas guiding unit 112 at the clearance distance C, a slit "S" is provided between the cover member 132 and each of the convex side walls 113', 113". These slits S still allow discharged air from the second gas discharge members 131, 131' to flow in laminar fashion over the convex side walls 113', 113" and pass through these slits S toward each of the at least one gas guiding members 12', 12", 12‴.

In an exemplary embodiment, the cover member 132 may have a height between 10 cm to 20 cm, *e.g.* 20 cm, and a width W_{c} of 3 cm to 7 cm, e.g. 5 cm.

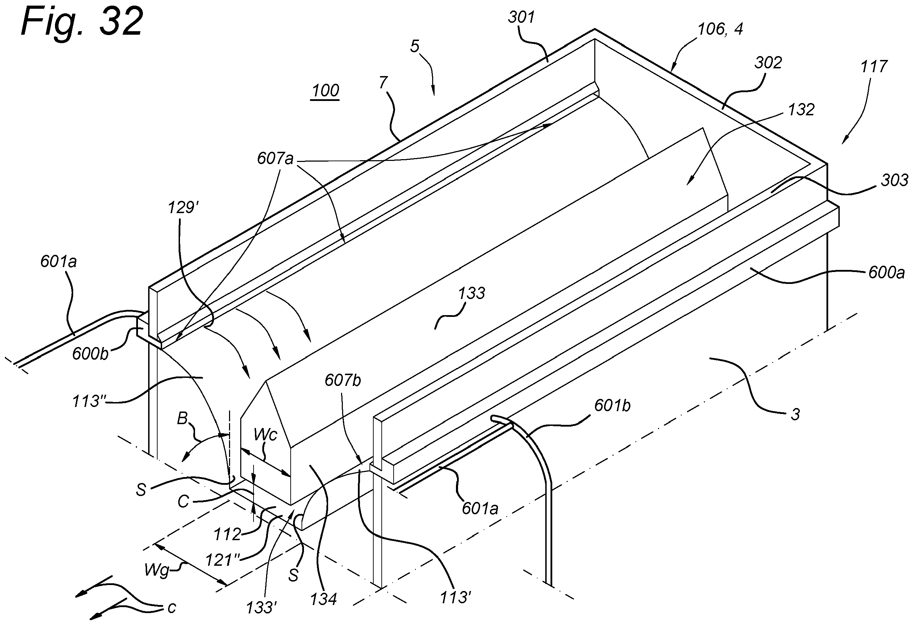

Figure 32 displays an embodiment of an insects transport device 100 with a similar set-up as the insects transport device 100 depicted in Figure 22, wherein in Figure 32 the further gas discharge members 131 and 131' located at the top side of the side walls in the embodiment of Figure 20 are now replaced by gas discharge members 600a and 600b, comprising elongated slits 607a and 607b respectively, for discharging gas, e.g. temperature and absolute humidity controlled air, in directions 129' over the convex surface of convex side walls 113', 113", similar to the embodiment of Figure 31. Again, by driving air over the convex surface, which air has controlled and set temperature and humidity, and in addition by controlling the air velocity by fan 603, with the insects transport device 100 displayed in Figure 31 and 32 it is now possible to better keep insects such as neonate black soldier fly larvae alive during their time of flight starting at the ovisite from which they hatch and ending in a crate 24K comprising larvae feed at a suitable humidity and temperature favorable for development of the living insects.

As mentioned earlier, the at least one reservoir 128, 128', 128a, 128a' comprising live insects, e.g. insect eggs or mites, are to be maintained at a controlled and predetermined temperature and relative air humidity to stimulate and facilitate optimal hatching or optimal disposal of mites through the through holes in the bottom floor of the mite cage 128a, 128a', such that optimal release of live insects from the at least one reservoir 128, 128', 128a, 128a' into the live insect receiving portion is achieved.

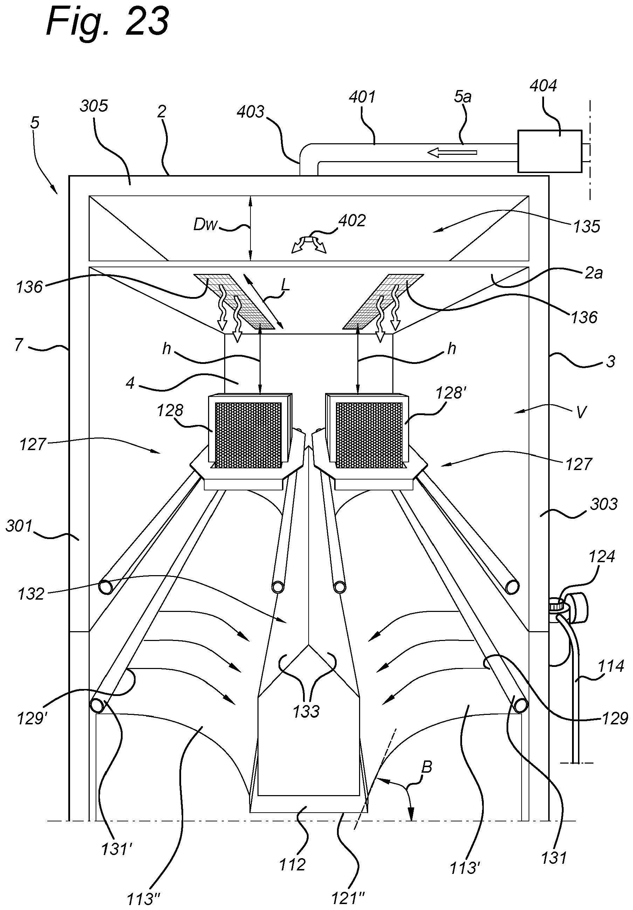

To provide optimal temperature and relative humidity condition, Figure 23 shows a casing 5 of an insects transport device 100 according to an embodiment. In the depicted embodiment, the insects transport device 100 comprises a thermally insulated casing 5 covering the gas guiding unit 112 in the inners side of the casing 5, the flat or convex side walls 113', 113", and the feeder arrangement 127 in which the at least one reservoirs 128, 128', 128a, 128a' are received. The casing 5 comprises a thermally insulated top wall 2 and thermally insulated side walls 3, 4, 4A, 7 defining the inner side, and in particular a closed inner space or volume "V" in which the temperature is controllable as well as the relative humidity to provide an environment for the at least one reservoir 128, 128', 128a, 128a' to stimulate and facilitate optimal hatching or to stimulate and facilitate optimal migration of mites through openings in the bottom floor of cages 128a, 128a'. In order to provide air of a particular temperature and/or relative humidity, the insects transport device 100 further comprises an air feed channel 5a, comprising tube 401 and connector 403 connected to the top wall 2 via opening 402 of the casing 5 for providing air of a desired temperature and/or relative humidity, under control of temperature control unit and relative air humidity control unit 404, to the inner side of the casing 5 and in particular to the inner volume V.

In an embodiment, the casing 5 may be provided with a secondary top wall 2a arranged below the top wall 2 at wall distance D_{w} therefrom such that a cavity space 135 is defined between the top wall 2 and secondary top wall 2a. The secondary top wall 2a further comprises one or more slits 136 such that air from the air feed conduit 5a entering the cavity/buffer space 135 is able to flow toward the inner volume V. That is, the one or more slits 136 fluidly connect the cavity/buffer space 135 and the inner volume V of the casing 5. The one or more slits 136 provided in the secondary top wall 2a allow air, e.g. temperature and/or humidity controlled air, to be provided to the inner volume V in distributed fashion so as to minimize turbulence in the inner volume. Therefore, the cavity space 135 in conjunction with the one or more slits 136 allow air from the air feed conduit 5a to enter the inner volume V with maximum homogeneity. The casing 5 is provided with thermally insulating top wall and side walls.

In an embodiment, the one or more slits 136 are arranged in longitudinal fashion, i.e. in a lengthwise direction "L" as depicted, thereby providing conditioned air in homogenous fashion along the gas guiding unit 112. In an exemplary embodiment, each of the one or more slits 136 extends along 70% to 90%, *e.g.* 80%, of a length of the first laminar flow of gas, e.g. air, over the top surface of the at least one gas guiding member 12', 12", 12‴. In an exemplary embodiment, each of the one more slits 136 has a length between 50 cm to 100 cm, e.g. 60 cm, 65 cm, 70 cm. In a further exemplary embodiment, each of the one or more slits 136 has a width of about 3 cm to 6 cm, e.g. 4 cm or 5 cm, to further facilitate homogenous distribution of conditioned air entering the inner volume V of the thermally insulated casing 5.

In an advantageous embodiment, the one or more slits 136 extend above the at least one reservoir 128, 128', 128a, 128a' containing the live insects, e.g. insect eggs or live mites, for which conditioned air is to be provided for optimized hatching, or optimized migration downward in the mite cage 128a, 128a'.

In another embodiment, each of the one or more slits 136 comprises a plurality of perforations covering 40% to 60%, *e.g.* 50%, of a surface area of the slit 136. In further embodiments each of the perforations is a substantially circular perforation having a diameter of about 4, 5, or 6 mm for example.

In an embodiment, the secondary top wall 2a with the one or more slits 136 is arranged above the at least one reservoir 128, 128' at a height of 5 cm to 15 cm, e.g. 10 cm to provide the conditioned air to the at least one reservoir 128. 128'.

As mentioned earlier, the insects transport device 100 may comprise a live insects counting device 8, *e.g.* a camera, for counting live insects in the first laminar flow exiting the insects transport device 100 at the proximal end of the live insect discharge member 11 as shown in Figures 13A, 1B, and 14. In one embodiment, the live insects discharge member 11 may be a funnel shaped discharge member 11, e.g. having a rectangular cross section, configured to provide a narrow stream of gas for accurate counting of the live insects exiting the insects transport device 100.

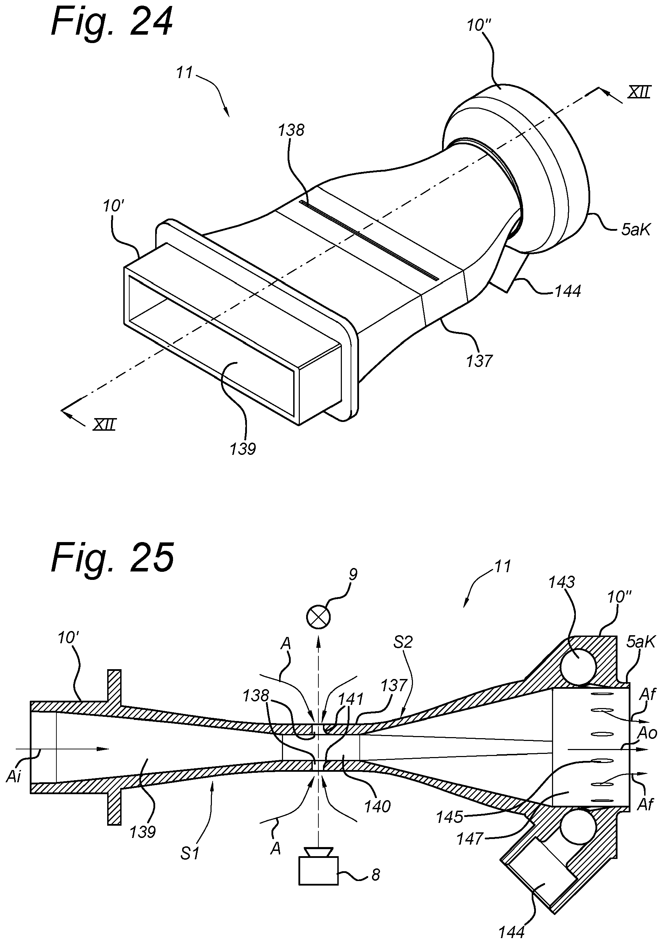

To further improve upon the accuracy and reliability of counting live insects exiting the insects transport device 100, further embodiments of the live insects discharge member 11 as discussed earlier are conceivable. For example, Figure 24 shows a three dimensional view of a live insect discharge member 11 and Figure 25 shows a cross sectional view of the live insect discharge member 11.

In the depicted embodiments, the live insect discharge member 11 may comprise a throat portion 137 arranged between the distal end 10', *i.e.* the first end, and a proximal end 10", *i.e.* the second end, of the live insect discharge member 11. That it, a discharge channel 139 of the live insect discharge member 11 extends between the distal end 10' and proximal end 10" thereof and comprises a constricted or choked channel portion 140 at the throat portion 137. Here, the distal/first end 10' is configured for connection to the insects transport device 100 such that live insects exiting the insects transport device 100 can travel through the discharge channel 139 by entering at the distal/first end 10' and exiting from the proximal/second end 10".

As shown, the throat portion 137 is provided with a through hole 138, e.g. shaped as a (elongated) slit 138, laterally/sideways extending through the throat portion 137. The through hole/slit 138 allows the counting device 3, *e.g.* a camera, to be arranged next to the slit shaped through hole 138 and have a field of view into the discharge channel 139, in particular the constricted channel portion 140, for counting the number of live insects passing through the live insect discharge member 11 as they exit the insects transport device 100.

The advantage of having the slit shaped through hole 138 at the constricted channel portion 140 is that a pressure drop in the constricted channel portion 140 will develop according to the Venturi effect or Venturi principle. That is, the constricted channel portion 140 induces a Venturi effect allowing outside air "A" to be drawn/sucked into the constricted channel portion 140 via the slit shaped through hole 138 when an air stream carrying live insects flows through the discharge channel 139. As a result, suction at the slit shaped through hole 138 allows live insects to be counted by the counting device 3 whilst preventing that live insects escape the live insect discharge member 11 via the slit shaped through hole 138.

For improved operation of the counting device 8, *e.g.* a camera, a light source such as a lamp 9 may be provided as mentioned earlier with reference to Figure 13A, 13B. To improve operation of the counting device 8, Figure 25 shows an embodiment of a light source 9 such as an elongated lamp arranged next to and extending along the slit shaped through hole 138 on an opposite side of the live insect discharge member 11 with respect to the counting device 8. In particular, the counting device 8 is arranged on a first side S₁ whereas the light source 9 is arranged on an opposing second side S₂ of the live insect discharge member 11. Light from the light source 9 is able to pass through the slit shaped through hole 138 and reach the counting device 8. The constricted channel portion 140 then prevents live insects escaping through the slit shaped through hole 138 by virtue of the suction effect explained above when an air stream carrying live insects passes through the discharge channel 139.

Note that suction at the slit shaped through hole 138 allows the counting device 3 to be arranged on both sides S₁, S₂, e.g. above or below, the live insect discharge channel 11 and the light source 9 may then be arranged below or above the live insect discharge channel 11 respectively. In any case, the constricted channel portion 140 prevents live insects escaping via the slit shaped through hole 138 on both sides S₁, S₂ of the live insect discharge member 11. Since live insects cannot escape through the slit shaped through hole 138, contamination of the counting device 8 and/or light source 9 is eliminated, allowing the counting device 8 and light source 9 to be placed on either side S₁, S₂ of the live insect discharge member 11 whilst still allowing accurate counting of the number of live insects exiting the insects transport device 100.

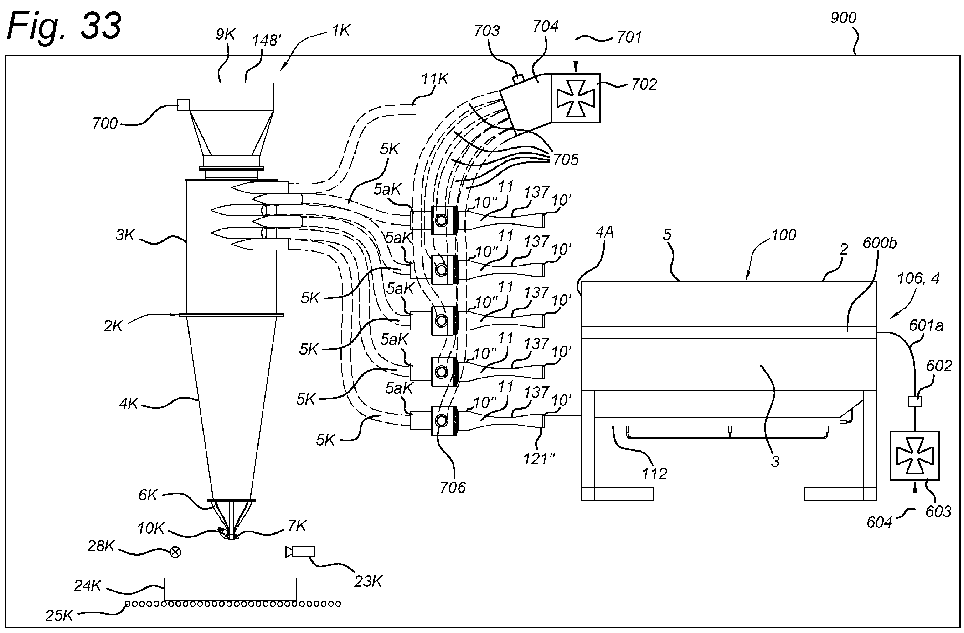

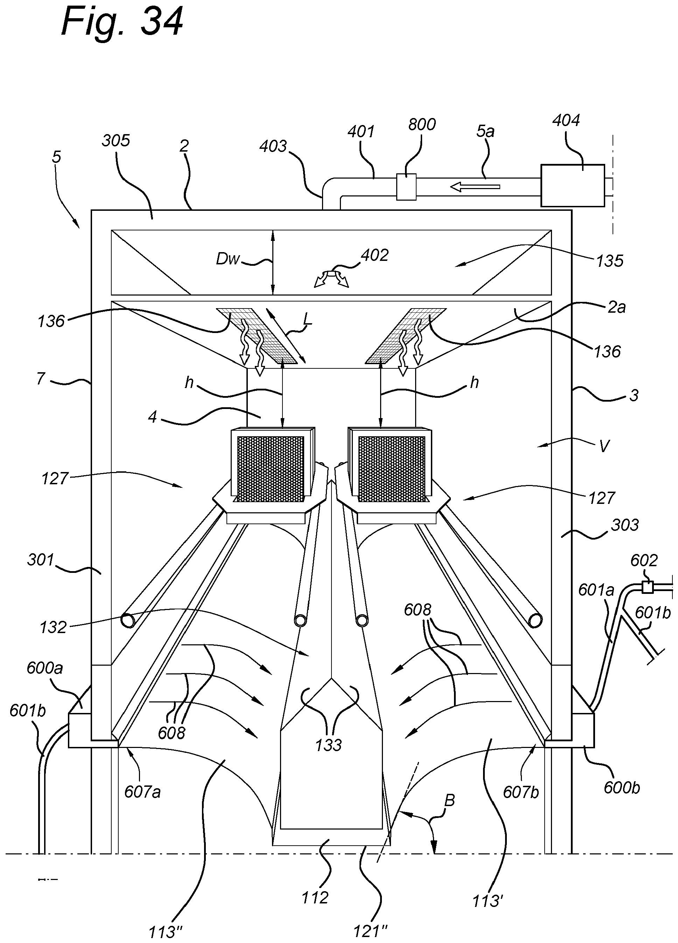

Figure 34 displays a casing 5 of an insects transport device 100 according to an embodiment similar to the embodiment outlined in Figure 24, with the difference that similar to the embodiments in Figures 31-33, wherein the further gas discharge members 131 and 131' located at the top side of the side walls in the embodiment of Figure 20 and Figure 24 are now replaced by gas discharge members 600a and 600b, comprising elongated slits 607a and 607b respectively, for discharging gas, e.g. temperature and absolute humidity controlled air, in directions 608 over the convex surface of convex side walls 113', 113". Gas discharge members 600a and 600b are connected to tubing or pipes 601a and 601b, respectively, jointly connected to driver 603 (See Figure 31 and Figure 33) such as a fan 603, which driver 603 drives ambient air through tubing or pipes 601a and 601b towards slits 607a and 607b. The air driven by fan 603 is temperature controlled air and absolute humidity or relative humidity controlled air. Temperature and humidity is controlled with sensor 602. The air temperature and air humidity is kept within temperature boundaries and within humidity boundaries suitable for keeping insect alive which are transported through the insect transport device 100 and cyclone separation system 1K.

As shown in Figure 24 and 25, in an embodiment the constricted channel portion 140 comprises a rectangular cross section, which allows a relatively narrow and elongated air stream of live insect to pass through the constricted channel portion 140 so that the counting device 8 is able to count the number of live insects much more accurately with a minimal number of uncounted live insects, which could have been be blocked by another live insect in the field of view of the counting device 8.

To obtain a most optimal field of view into the constricted channel portion 140, an embodiment is provided wherein the slit shaped through hole 138 has a length of at least 90% percent of a width of the constricted channel portion 140 in the lateral direction of the slit shaped through hole 138. This embodiment minimizes the number of live insects that could potentially bypass the field of view of the counting device 8.

In an embodiment, the slit shaped through hole 138 comprises a chamfered or rounded downstream inner edge 141, i.e. extending in the lengthwise direction of the slit shaped through hole 138 on a downstream side thereof, thereby reducing turbulence and maintaining laminar flow within the constricted channel portion 140 when air A is being drawn into the constricted channel portion 140 in the direction of air flowing from the first end 10' to the second end 10".

The live insect discharge member 11 with the slit shaped through hole 138 enabling a field of view into the constricted channel portion 140 allows for an extremely useful counting device 8 which is able to accurately count the number of live insects exiting the insects transport device 100. In particular, because accurate counting of live insects is now possible by means of the live insect discharge member 11, information on hatch and development characteristics of live insects in the insects transport device 100 can be deduced. For example, by counting the number live insects passing the live insect discharge member 11 it is possible to deduce what the effects are of temperature and /or relative humidity on live insects (e.g. insect eggs, mature mites) and their hatch time (e.g. when eggs of for example black soldier flies are present in ovisites 128, 128') or their migration time (e.g. when mites are present in the reservoir(s) 128a, 128a') in the at least one reservoir 128, 128a. Therefore, the live insect discharge member 11 and counting device 8 allow for gaining further information on live insect hatching characteristics or live insect migration characteristics.

Although the constricted channel portion 140 prevents live insect escaping though the slit shaped through bore 138, an outgoing air stream Aₒ with live insects exiting the live insect discharge member 11 at its proximal/second end 10" is generally slower than an incoming air stream Aᵢ entering the distal/first end 10'. To compensate for this loss of speed, an embodiment is provided wherein the proximal/second end 10" of the live insect discharge member 11 is provided with an air amplifier unit 5aK which is configured to inject further air Af into the second end 10" of the live insect discharge member 11. This ensures that an outgoing air stream Aₒ with live insects has sufficient speed and momentum to flow to other parts of the insects transport device, such as a cyclone separation system 1K, connected to the second end 10" of the live insect discharge member 11.

In an exemplary embodiment, the air amplifier unit 5aK comprises a circumferential chamber 143 fluidly coupled to an air feed connection 144 for connection to an air feed allowing further air A_{f} to be injected into the proximal second end 10" of the live insect discharge member 11, and wherein one or more air amplifier outlets 145 are circumferentially arranged in an inner wall 147 of the second end 10" of the live insect discharge member 11 and wherein the one or more air amplifier outlets 145 are fluidly connected to the circumferential chamber 143. In this embodiment, the one or more air amplifier outlets 145 allow for an even injection of the further air Af into the second end 10" such that turbulence is minimised. In an exemplary embodiment, a single air amplifier outlet 145 may be provided in the form of a circumferential slit in the inner wall 147 fluidly coupled to the circumferential chamber 143, allowing for even injecting of further Af.

As mentioned above, the air amplifier unit 5aK allows for an outgoing air stream Aₒ with live insects which has sufficient speed and momentum to flow to other parts of a system, such as a cyclone separator 1K, connected to the second end 10" of the live insect discharge member 11.

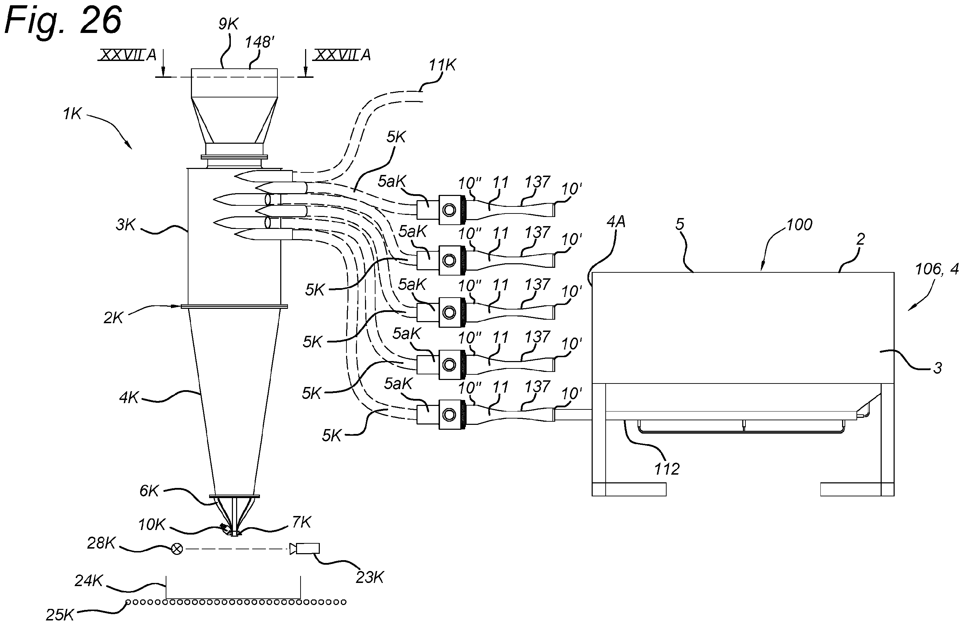

Figure 26 shows a cross sectional view of such a cyclone separation system 1K connected to one or more insects transport devices 100 according to an embodiment. In the embodiment shown, the transport device 100 comprises the live insect discharge member 11 described earlier, e.g. comprising the throat portion 137 with the slit shaped through hole 138 and the constricted channel portion 140 to prevent live insects escaping there through by virtue of the Venturi effect. A counting device 8 may be provided next to the slit shaped through hole 138, possibly with a light source 9 such as a lamp on an opposite side of the throat portion 137. The slit shaped through hole 138 allows the counting device 8 to have a field of view into the constricted channel portion 140 for counting live insects passing through the live insect discharge member 11. The light source 9 is able to provide additional illumination through the slit shaped through hole 138.

As depicted, a cyclone separation system 1K is connected to one or more insects transport devices 100 to separate live insects from an outgoing air stream Aₒ of each live insect discharge member 11. The cyclone separation system 1K comprises a main cyclone chamber 2K having a top chamber part 3K and a conical shaped bottom chamber part 4K, wherein the top chamber part 3K is connected to one or more intake channels 5K each of which is arranged for connection to a primary air source providing an air stream comprising live insects. Here, the air stream provided by the primary air source is an outgoing air stream Aₒ of a live insect discharge member 11 as described above. Therefore, each of the one or more intake channels 5K is arranged for connection to an insects transport device 100 of the one or more insects larvae transport devices 100.

Note that only one insects larvae transport device 100 is depicted for clarity purposes and the skilled person will understand the each of the depicted first ends 10' of the live insect discharge members 11 is connected to an insects transport device 100.

The bottom chamber part 4K of the cyclone separation system 1K is connected to a discharge nozzle 6K comprising a discharge end having a main discharge conduit (not shown) for discharging the live insects from the cyclone separation system 1K. The discharge end comprises an air injection member 7K for connection to a secondary air source 10K and wherein the air injection member 7K is configured to inject air back into the discharge nozzle 6K. Injecting air back into the discharge nozzle 6K stops the discharge of live insects.

In an advantageous embodiment, the air injection member 7K is configured for intermittent air injection back into the discharge nozzle 6K.

Each of the one or more insects transport devices 100 provides an outgoing air stream Aₒ with live insects passing through a live insect discharge member 11 toward the cyclone separation system 1K, which subsequently discharges separated live insects in batch wise fashion by intermitted operation of the air injection member 7K. When desired, the cyclone separation system 1K, discharges separated live insects in continuous fashion by continuous operation of the air injection member 7K.

As the skilled person will understand, in operation the one or more intake channels 5K carrying the outgoing air streams Aₒ induce a main vortex in the top chamber part 3K allowing centrifugal separation of the live insects from the combined outgoing air streams Aₒ in the top chamber part 3K. The separated live insects follow a conical inner wall of the bottom chamber part 4K toward the discharge nozzle 6K. Due to the conical shaped bottom chamber part 4K, an ascending inner vortex of "clean" air is generated that exits the top chamber part 3K through an air exit 9K arrange thereon.

Discharged live insects may be collected in a container 24K arranged underneath the discharge nozzle 6K and wherein the container 24K is movable by means of a conveyor system 25K. For example, such container is a crate provided with feed substrate for live insects such as insect larvae, such as for example neonate larvae of black soldier fly. For example, in case the container 24K contains a desired number of live insects, then the air injection member 7K may be activated to inject air back into the discharge nozzle 6K as a result of which discharge of live insects is temporarily stopped. As the discharge of live insects has stopped, the container 24K may be replaced with another container, and once the other container has been correctly positioned, the air injection member 7K may be deactivated to resume discharge of separated live insects from the cyclone separation system 1K. This way, accurate, controllable and constant dosing of for example live adult insects such as live mites is made possible.

In an embodiment, the cyclone separation system 1K may comprise a further counting device 23K, e.g. a further camera, arranged next to the discharge nozzle 6K for counting the number of live insects being discharged therefrom. Activation and deactivation of the air injection member 7K may be controlled based on the counted number of live insects being discharged. Optionally, a further light source 28K may be provided to improve illumination conditions for the further counting device 23K.

As further shown, the second end 10" of each live insect discharge member 11 may be provided with an air amplifier unit 5aK to boost the outgoing air stream Aₒ such that it attains sufficient speed and momentum.

Advantageously, a plurality of insects transport devices 100 are connected to a corresponding number of intake channels 5K so that the cyclone separation system 1K may operate continuously without interruption to the flow of live insects entering the cyclone separation system 1K. In this way the cyclone separation system 1K can be scaled up to achieve batch wise discharge of any desired number of live insects. Note that the top chamber part 3K may be connected to an auxiliary intake channel 11K configured to provide a "pilot" air stream into the top chamber part 3K to further optimize centrifugal separation of the live insects entering the main cyclone body 2K.

These embodiments of insects transport devices of the invention are all suitable for transportation of live neonate larvae of the black soldier fly, which larvae have a body diameter of between 1 mm and 4 mm and a body length which ranges between 5 mm and 12 mm. In addition, these embodiments of insects transport devices of the invention are all suitable for transportation of live insects such as mites.

While the invention has been described in terms of several embodiments, it is contemplated that alternatives, modifications, permutations and equivalents thereof will become apparent to one having ordinary skill in the art upon reading the specification and upon study of the drawings. The invention is not limited in any way to the illustrated embodiments. Changes can be made without departing from the scope which is defined by the appended claims.

Figure 33 shows a cross sectional view of such a cyclone separation system 1K connected to one or more insects transport devices 100 according to an embodiment similar to the embodiment outlined in Figure 26. In the embodiment of Figure 33, the transport device 100 comprises the gas discharge members 600a and 600b, comprising elongated slits 607a and 607b respectively, for discharging gas, e.g. temperature and absolute humidity controlled air, in directions 129' over the convex surface of convex side walls 113', 113", similar to the embodiment of Figure 31 and 32. Again, by driving air over the convex surface, which air has controlled and set temperature and humidity, and in addition by controlling the air velocity by fan 603, with the insects transport device 100 displayed in Figure 31 and 32 it is now possible to better keep insects such as neonate black soldier fly larvae alive during their time of flight starting at the ovisite from which they hatch and ending in a crate 24K comprising larvae feed at a suitable humidity and temperature favorable for development of the living insects. The air amplifier unit 5aK of each of the insects transport device 100 now comprised by the cyclone separation system 1K is in this embodiment connected through connectors 706 to a tube or a pipe 705, which tubes or pipes 705 are connected to a driver such as a fan through connector 704 provided with an air temperature control unit 703 and absolute air humidity control unit 703, for controlling the temperature and air humidity of the (ambient) air 701 driven by fan 702 through pipes 705 towards air amplifiers 5aK. This way, temperature and air humidity of the air applied for amplifying the air stream blown from the direction of the insects transport device 100 towards the cyclone top chamber part 3K and comprising living insects such as neonate larvae, is kept within temperature boundaries and absolute air humidity boundaries favourable for keeping transported insects alive, and at the same time keeping these insects from touching walls or inner sides of tubes, *etc.,* and preventing insects from sticking to sides of inner pipes, tubes, cyclone chambers, *etc.* Preferably, the cyclone separation system 1K and the cyclone separation system 1K comprising one or more insects transport devices 100 and the insects transport devices 100 are kept in an air-conditioned room. Preferably in the air-conditioned room, air temperature and air absolute humidity are such that when this air is provided by fan 702 and/or fan 603 inside the cyclone separation system 1K at an air velocity suitable for transporting living larvae and for keeping the larvae alive and air born, the air temperature and the air humidity contribute to the health of the insects and aids in keeping the insects alive during transport, counting and dosing.

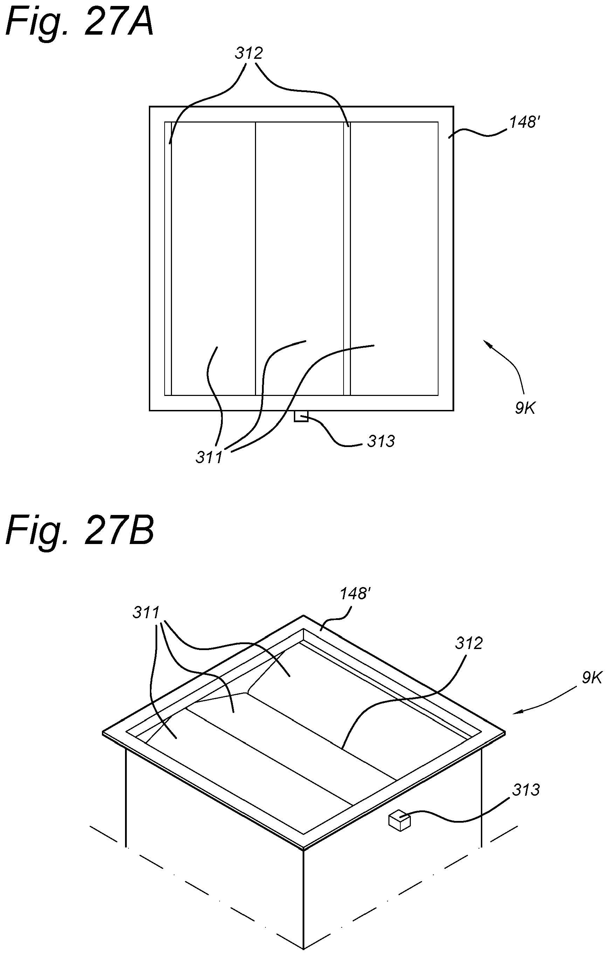

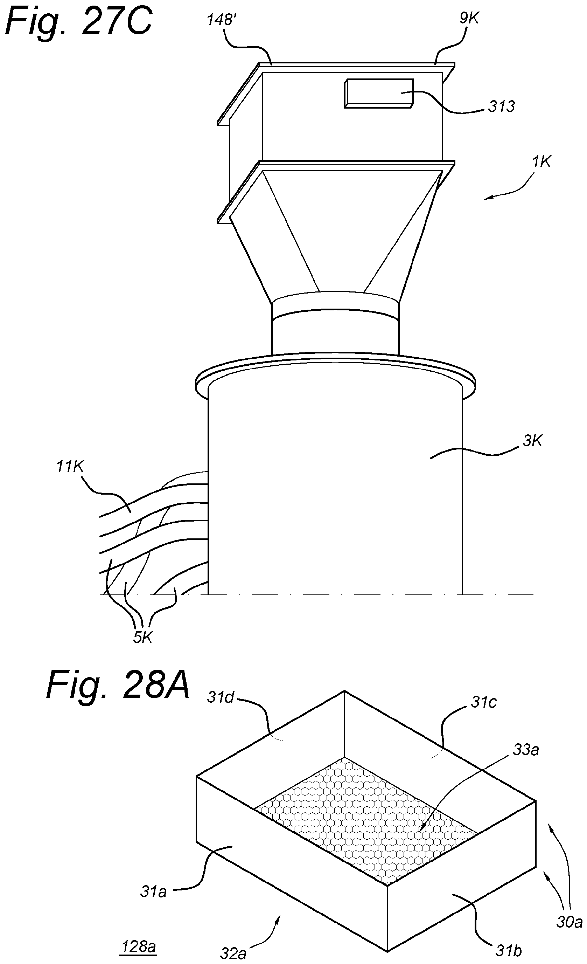

Turning to Figure 27A, the top view of the cyclone separation system 1K is shown, the cyclone separation system comprised by the insects transport device of the invention, wherein the top view shows laminar slats 311 that are openable under control of a control unit 313. The slats are pivotally connected to upper portion 148' of the cyclone separation system, through pivots 312. Operating the slats 311 provides the possibility to adjust and for example temporarily increase the air pressure inside the cyclone separation system independently of the contribution to the air pressure by the transport air entering the cyclone separation system from the live insects discharge member, by partly or wholly shutting the laminar slats. Figure 27B shows a perspective top/side view of the cyclone separation system 1K, comprised by the insects transport device of the invention, showing laminar slats in the top portion 148' of the system 1K and Figure 27C shows a side view of part of the cyclone separation system 1K. By providing the cyclone separation system with these laminar slats, the operation of the insects transport device in so far the laminar flow of air is considered, is independent of the operation of the insects transport device in so far the batch wise dosing of live insects by use of the cyclone separation system is considered. Thus, air pressure and air flow velocity with regard to the laminar air flow inside the casing 5, 105, is controllable and adjustable without influencing the live insects dosing operation of the cyclone separation system part of the insects transport device.

In view of the above, an embodiment may thus be provided wherein the cyclone separation system 1K comprises an air exit 9K arranged on the top chamber part 3K and wherein the air exit 9K comprises pivotally arranged slats 311, e.g. openable slats 311 with pivots 312, thereby allowing adjustment of air pressure inside the cyclone separation system 1K. In an even further embodiment, the air exit 9K may comprise a slat operation driver/control unit 313 for moving the slats 311 between an open state and a closed state.

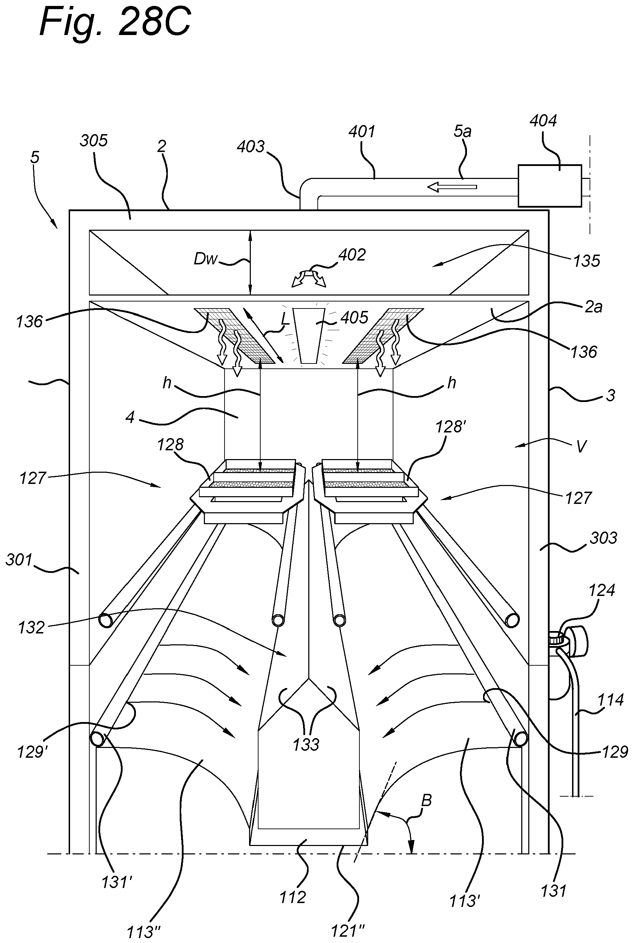

The live insects device of the invention provides for efficient and accurate and constant dosing of live insects such as insect eggs, embryo, neonate larvae, larvae, prepupae, pupae, imago, adult insect, for example fly neonate larvae such as black soldier fly larvae 1 second - 1 day of age, preferably 10 seconds - 2 hours of age, or for example imago such as mites. For applying the insects transport device 1, 100 for counting, dosing such as batch wise dosing, of e.g. imago such as mites, a reservoir 128a adapted to the delivery of such mites to the laminar air flow, is provided. Figure 28A shows a reservoir 128a, consisting of a cage 128a for live insects such as mite, the cage 128a comprising side walls 31a-31d and a bottom floor 32a comprising openings 33a for passage of live insects. The openings in the bottom floor 32a of the cage 128a are typically provides as through holes 33a, slits 33a, a mesh 33a, a sieve 33a, *etc.,* wherein the openings have dimensions suitable for passage of live insects at the desired stage and age of their development, such as adult mites. Figure 28B displays an inside view of an insects transport device 1, 100 of the invention. Shown are longitudinal gas transport members 12', 12" which are connected imbricatedly at positions 21, 22 and 21', 22'. Where two consecutive gas transport members are coupled imbricatedly, a gas discharge member (See 20, 20' in Figure 15 and 114', 114", 114‴ in Figure 18) is positioned at the location where said gas transport members overlap, said gas discharge member provided with openings 23, 23' for discharging gas. The insects transport device 1, 100 comprises a reservoir 128a, *i.e.* a cage 128a for keeping mites, the cage 128a comprising side walls 31a-31d and a bottom floor 32a comprising openings 33a for passage of live insects. The cage 128a is supported by support member 30a, *i.e.* a frame 30a for receiving the cage 128a. A further frame, 30a' for receiving a further cage (reservoir) 128a' is also displayed. Figure 28C shows a thermally insulated casing 5 of an insects transport device 100 according to an embodiment of the present invention, the insects transport device comprising a reservoir 128a, the reservoir being a cage 128a for live insects, such as imago, such as mites, the cage 128a, 128a' comprising side walls 31a-d and a bottom floor 32a comprising openings 33a for passage of live insects, the casing 5 comprising a secondary top wall 2a defining a volume 135.

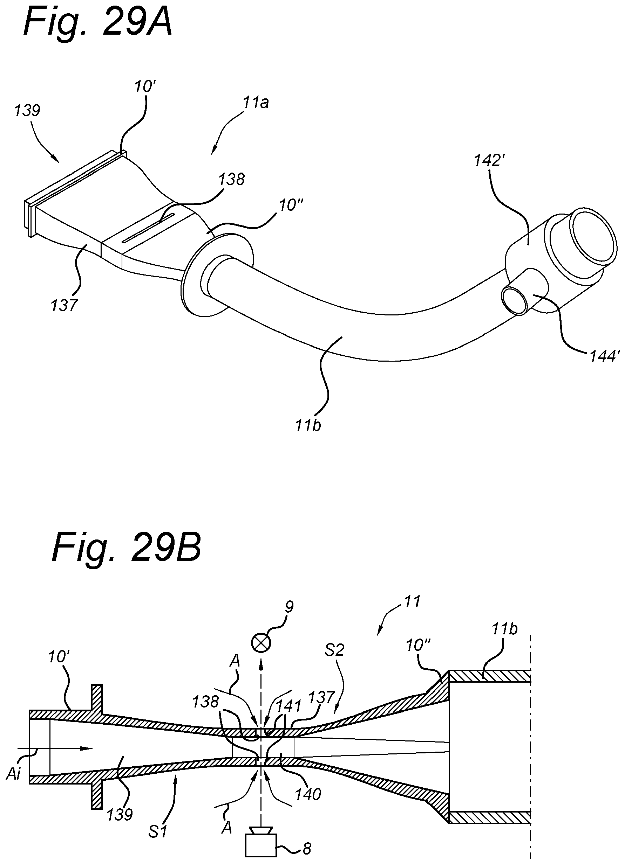

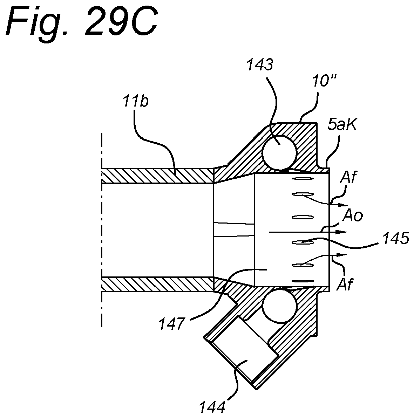

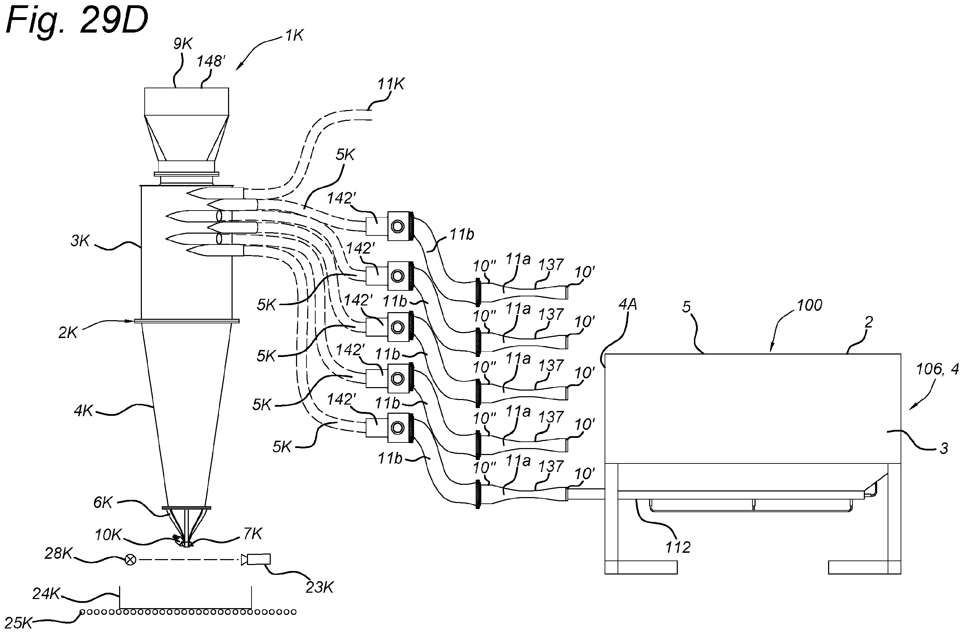

Figure 29A displays an insect discharge member 11a coupled to a tube 11b, the tube 11b connected to an air amplifier unit 142'. Figure 29B displays a cross-sectional side view of the insect discharge member 11a connected to tube 11b displayed in Figure 29A. Figure 29C shows a cross-sectional side view of air amplifier unit 142' displayed in Figure 29A, fluidly connected to tube 11b, which is connected at its proximal end to the insect discharge member 11a as displayed in Figure 29B. Figure 29D shows a schematic view of an insects transport device 100 further provided with a cyclone separation system 1K fluidly connected to the live insect discharge member 11a via tubing 11b and air amplifier unit 142', according to an embodiment of the present invention.

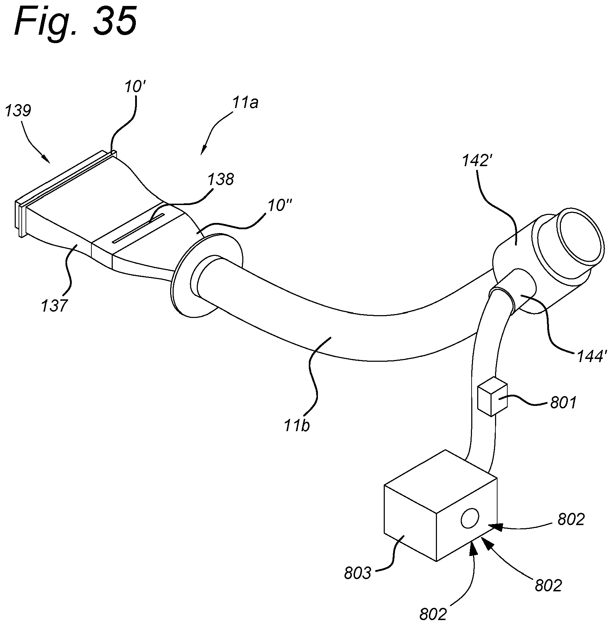

Figure 35 shows an insect discharge member 11a coupled to a tube 11b, the tube 11b connected to an air amplifier unit 142', similar to the insects discharge member 11a of as outlined in Figure 29A, though with the additional driver 803 such as a fan 803, for driving gas such as ambient air 802 towards connector 144' which connects the fan with air amplifier 142'. Sensor 801 senses and/or controls the temperature and air humidity of the air 802 driven by driver 803 towards the air amplifier 142' and into the cyclone separation system 1K.

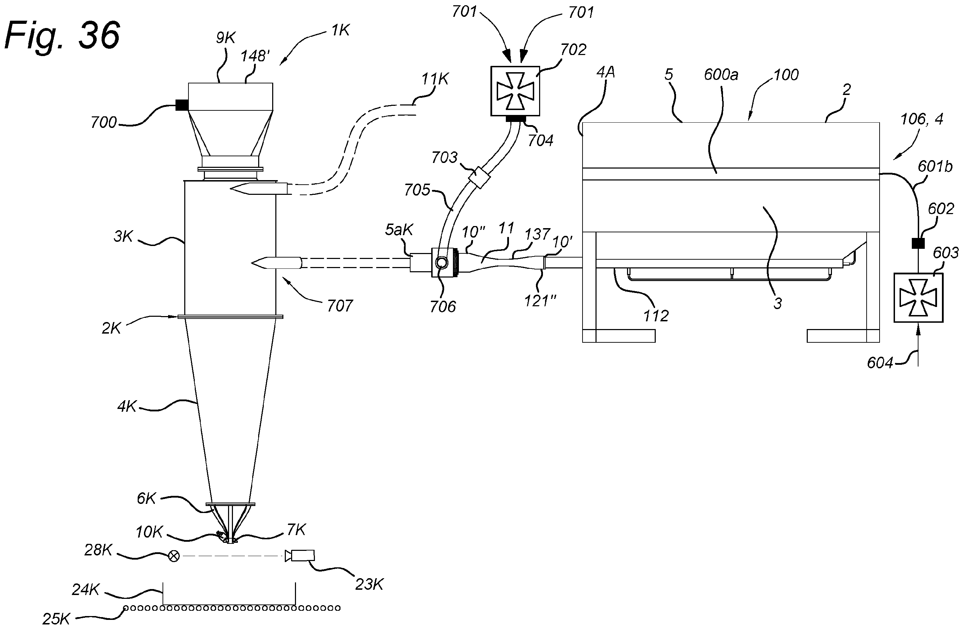

Similar to the cyclone separation system 1K of the embodiment displayed in Figure 29D, Figure 36 shows a schematic view of a cyclone separation system 1K further provided with an insects transport device 100 fluidly connected to the live insect discharge member 11a via tubing 11b and air amplifier unit 142', according to an embodiment of the present invention. The embodiment of Figure 36 differs from the embodiment in Figure 29D in that the cyclone portion encompassing top cyclone chamber 3K comprising connector 707 for connecting insects transport device 100 to the cyclone chamber 3K which is at the same height relative to the horizontal as the proximal end 121" of the gas guiding unit 112. Herewith, living insects such as mites and black soldier fly larvae are transported through essentially horizontally oriented tubing or pipes, preferably rigid pipes from the insects transport device 100 portion to and into upper cyclone chamber 3K of the cyclone separation system 1K. This way, the risk and chance for insects hitting internal side walls of tubing, pipes, *etc.* is further lowered. Moreover, with straight tubing and pipes, risk for air turbulence inside the tubing and pipes is reduced or even absent such that air borne transported living insects are prevented from being blocked, blown to inner walls, accumulation in certain spots of the system, *etc.*

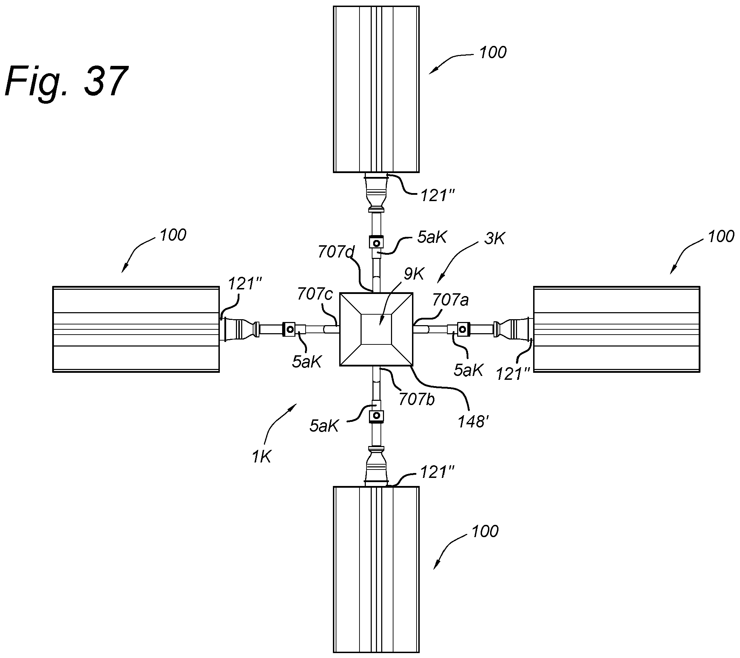

The embodiment displayed in Figure 37 resembles the cyclone separation system 1K displayed in Figure 37, with now four insects transport devices 100 coupled the proximal end 121" of the gas guiding units 112 (see Figure 36) to the upper cyclone chamber 3K through connectors 707a-d. Herewith, these connection points provided by connectors 707a-d for connecting the insects transport devices 100 to the upper cyclone chamber 3K, and the proximal ends 121" of the gas guiding units 112 are essentially at the same height relative to the horizontal. By distributing the insects transport devices at more or less equal distances around the central cyclone chamber portion of the cyclone separation device 1K, for example for four insects transport devices 100 in more or less an East, South, West, North orientation, the pipes and/or tubes connecting the cyclone chamber with the insects transport devices are essentially in horizontal orientation. As said, this horizontal orientation aids in smooth unhampered transport of air-borne living insects and contributes to keeping insects alive during transport since sticking to inner walls and bumping to inner sides is prevented.

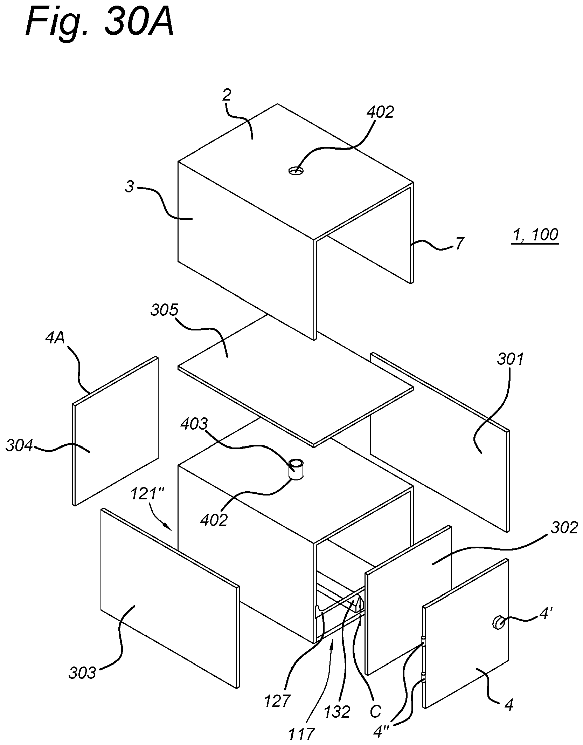

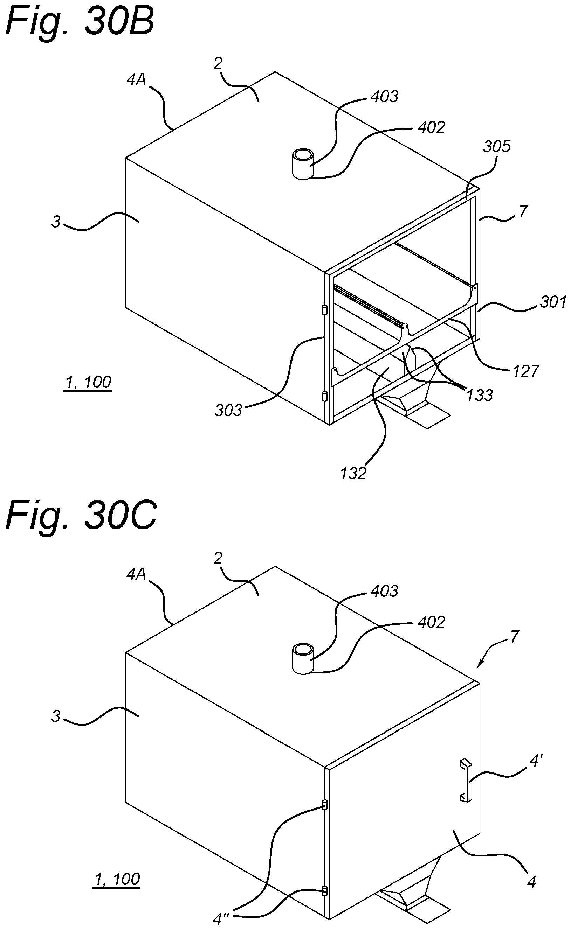

Figure 30A displays an exploded view of an insects transport device 1, 100, showing the side walls 3, 4, 4A, 7 and top wall 2 of the casing 5, 105, said side walls 3, 4, 4A, 7 and top wall 2a provided with a layer 303, 302, 304, 301, 305 of thermally insulating material respectively, wherein the side wall 4 is an openable door 4 provided with a knob or grip 4' and pivots 4". Figure 30B displays an insects transport device 1, 100 provided with casing 5, 105, wherein said casing comprises thermally insulated side walls 3, 4, 4A, 7 and an thermally insulated top wall 2. For clarity the front side wall 4 is not shown. For side walls 3 and 7 and for top wall 2, the layers of thermally insulating material 301, 303 and 305 are visualized. The feeder arrangement inside the casing is visible, as well as the cover member 132 inside the casing. In the top wall 2 of the casing, through hole 402 is visualized, together with connector 403, which is part of the air feed channel 5a (see Figure 23 and Figure 28C). Figure 30C displays an insects transport device 1, 100 provided with casing 5, 105, wherein said casing comprises thermally insulated side walls 2, 3, 4, 4A and a thermally insulated top wall 2, according to an embodiment of the invention. Side wall 4 is an openable door 4 provided with a grip 4' and pivots 4". The top wall 2 of the casing comprised by the insects transport device comprises opening 402 for receiving the connector portion 403 of the air feed channel 5a.

## Claims

1. A cyclone separation system (1K) for separating live insects carried by an air stream, comprising:
a main cyclone chamber (2K) having a top chamber part (3K) and a conical shaped bottom chamber part (4K), wherein the top chamber part (3K) is connected to one or more intake channels (5K) each of which is arranged for connection to a primary air source providing an air stream (AK) comprising live insects, and wherein the bottom chamber part (4K) is connected to a discharge nozzle (6K) comprising a discharge end (7K) having a main discharge conduit (8K) for discharging the live insects from the cyclone separation system (1K), and
**characterized in that**
the discharge end (7K) comprises an air injection member (10K) for connection to a secondary air source and wherein the air injection member (10K) is configured to inject air back into the discharge nozzle (6K) for stopping the discharge of the separated live insects.

2. The cyclone separation system according to claim 1, wherein the air injection member (10K) of the discharge end (7K) comprises a first air chamber (15K) and a first air injection conduit (16K) fluidly connecting the first air chamber (15K) and the main discharge conduit (8K) of the discharge end (7K), wherein the first air injection conduit (16K) is arranged to provide a first injected air flow (F1K) in a direction back into the discharge nozzle (6K).

3. The cyclone separation system according to claim 2, wherein the first air injection conduit (16K) is arranged at a first injection angle (α₁K) smaller than 60° degrees with respect to a longitudinal axis (LK) of the discharge nozzle (6K),
preferably, wherein the first injection angle (α₁K) lies between 40° and 60° degrees with respect to the longitudinal axis (LK) of the discharge nozzle (6K), e.g. wherein the first injection angle (α₁K) is about 45° degrees.

4. The cyclone separation system according to claim 3, wherein the discharge nozzle (6K) comprises a first inner wall portion (17K) which is arranged at a first wall angle (β₁K) with respect to the longitudinal axis (LK) of the discharge nozzle (6K), and wherein the first injection angle (α₁K) of the first air injection conduit (16) is substantially aligned with/equal to the first wall angle (β₁K).

5. The cyclone separation system according to any one of claims 2-4, wherein the first air injection conduit (16K) is a slit shaped conduit extending in lateral direction between the first air chamber (15K) and a first discharge conduit wall portion (18K) of the main discharge conduit (8K), preferably, wherein the slit shaped first air injection conduit (16K) has a width (W₁K) of about 0.2 mm to 1 mm,
more preferably wherein the slit shaped first air injection conduit (16K) has a length (LsK) of more than 50% of a width (WcK) of the main discharge conduit (8K), e.g. more than 75%, e.g. more than 90%, e.g. more than 95%, e.g. 100% of the width (WcK).

6. The cyclone separation system according to any one of claims 2-5, wherein the air injection member (10K) of the discharge end (7K) comprises a second air chamber (19K) and a second air injection conduit (20K) fluidly connecting the second air chamber (19K) and the main discharge conduit (8K) of the discharge end (7K), wherein the second air injection conduit (20K) is arranged to provide a second injected air flow (F2K) in a direction back into the discharge nozzle (6K).

7. The cyclone separation system according to claim 6, wherein the second air injection conduit (20K) is arranged at a second injection angle (α₂K) smaller than 60° degrees with respect to a longitudinal axis (LK) of the discharge nozzle (6K).

8. The cyclone separation system according to claim 7, wherein the second injection angle (α₂K) lies between 40° and 60° degrees with respect to the longitudinal axis (LK) of the discharge nozzle (6K), e.g. wherein the second injection angle (α₂K) is about 45° degrees.

9. The cyclone separation system according to claim 7, wherein the discharge nozzle (6K) comprises a second inner wall portion (21K) being arranged at a second wall angle (β₂K) with respect to the longitudinal axis (LK) of the discharge nozzle (6K), and wherein the second injection angle (α₂K) of the second air injection conduit (20K) is substantially aligned with the second wall angle (β₂K).

10. The cyclone separation system according to any one of claims 6-9, wherein the second air injection conduit (20K) is a slit shaped conduit extending between the second air chamber (19K) and a second discharge conduit wall portion (22K) of the main discharge conduit (8K),
preferably, wherein the slit shaped second air injection conduit (20K) has a width (W₂K) of about 0.2 mm to 1 mm.

11. The cyclone separation system according to any one of claims 6-10, wherein the first and second air injection conduits (16K, 20K) are arranged on opposite sides of the main discharge conduit (8K).

12. The cyclone separation system according to claim 11, wherein the first and second air injection conduits (16K, 20K) are laterally/sideways offset in opposite direction,
preferably, when depending from claims 5 and 10, wherein the slit shaped first and second air injection conduits (16K, 20K) each have a length (LsK) of at most 50% of a width (WcK) of the main discharge conduit (8K).

13. The cyclone separation system according to claim 11, wherein the first air injection conduit (16K) comprises a plurality of first conduit sections (16aK) and wherein the second air injection conduit (20K) comprises a plurality of second conduit sections (20aK), wherein the plurality of first and second conduit sections (16aK, 20aK) are laterally/sideways offset in alternating manner along a width (WcK) of the main discharge conduit (8K).

14. The cyclone separation system according to any one of claims 1-13, when depending from claim 2 and 8, wherein the first and second air chambers (15K, 19K) are arranged on opposite sides of the main discharge conduit (8K) and are fluidly connected to one another.

15. The cyclone separation system according to any one of claims 1-14, wherein an intake end (12K) of the discharge nozzle (6K) is circular and wherein the main discharge conduit (8K) of the discharge nozzle (6K) is substantially rectangular,
preferably, when depending from claim 2, wherein the air injection member (10K) of the discharge end (7K) comprises two opposing auxiliary air chambers (30K) and two opposing auxiliary injection conduits (29K) each of which fluidly connects one of the two auxiliary air chambers (30K) with the main discharge conduit (8K) of the discharge end (7K),
wherein each auxiliary injection conduit (29K) is arranged to provide an auxiliary injected air flow (G1K, G2K) in a direction back into the discharge nozzle (6K), and
wherein the two opposing auxiliary air chambers (30K) and the two opposing auxiliary injection conduits (29K) are arranged along shortest sides (SsK) of the substantially rectangular shaped main discharge conduit (8K).

16. The cyclone separation system according to any of claims 1-15, further comprising a camera based counting system (23k) arranged at the discharge end (7K) of the discharge nozzle (6K),
preferably, wherein the camera based counting system (23K) defines a planar triangular field of view (FVK) and wherein the main discharge conduit (8K) comprises a trapezium shaped cross section having two opposing non-parallel sides (25K, 26K) each of which is parallel to an edge (27K) of the planar triangular field of view (FVK).

17. The cyclone separation system according to any of claims 1-16, wherein each of the intake channels (5K) comprises an air amplifier unit (5aK) for providing a supplementary air stream to the air stream (AK) in a flow direction thereof.

18. The cyclone separation system according to any one of claims 1-17, wherein the discharge nozzle (6K) further comprises a discharge guiding member (32K) mounted to the discharge end (7K) of the discharge nozzle (6K),
wherein the discharge guiding member (32K) comprises an expanding guiding channel (33K) fluidly coupled to the main discharge conduit (8K) for receiving live insects when the cyclone separation system (1K) is in operation,
preferably, wherein the discharge nozzle (6K) comprises a laterally extending elongated opening (34K) arranged between the discharge guiding member (32K) and the discharge end (7K), and wherein the laterally extending elongated opening (34K) further extends parallel along the main discharge conduit (8K) and along an opening width (WoK) equal to or larger than a width (WcK) of the main discharge conduit (8K),
more preferably, wherein the discharge guiding member (32K) may further comprise a lower circumferential rim portion (35K), e.g. a circumferential flange portion, configured for engagement with a circumferential rim portion (24aK) of a container (24K).

19. A method of providing batches of live insects, comprising
a) providing a cyclone separation system (1K) according to any of claims 1-18;
b) connecting each of the one or more intake channels (5K) to a primary air source providing an air stream (AK) comprising live insects and connecting the air injection member (10K) to a secondary air source;
c) collecting separated live insects being discharged from the discharge nozzle (6K); and when a desired number of live insects have been collected,
d) injecting air back into the discharge nozzle (6K) with the air injection member (10K) for a predetermined time period to temporarily stop discharge of live insects from the discharge nozzle (6K); and during the predetermined time period,
e) transferring the collected live insects away from the discharge nozzle (6K).

## Patentansprüche

1. Zyklonabscheidersystem (1K) zum Abscheiden von lebenden Insekten, die von einem Luftstrom getragen werden, aufweisend:
eine Hauptzyklonkammer (2K), die einen oberen Kammerteil (3K) und einen konisch geformten unteren Kammerteil (4K) aufweist, wobei der obere Kammerteil (3K) mit einem oder mehreren Einlasskanälen (5K) verbunden ist, von denen jeder zur Verbindung mit einer Primärluftquelle angeordnet ist, die einen Luftstrom (AK) bereitstellt, der lebende Insekten aufweist, und wobei der untere Kammerteil (4K) mit einer Abführdüse (6K) verbunden ist, die ein Abführende (7K) aufweist, das einen Hauptabführkanal (8K) zum Abführen der lebenden Insekten aus dem Zyklonabscheidersystem (1K) hat, und
**dadurch gekennzeichnet, dass**
das Abführende (7K) ein Lufteinblasbauteil (10K) zur Verbindung mit einer Sekundärluftquelle aufweist und wobei das Lufteinblasbauteil (10K) so gestaltet ist, dass es Luft zurück in die Abführdüse (6K) einbläst, um das Abführen der abgeschiedenen lebenden Insekten zu unterbinden.

2. Zyklonabscheidersystem nach Anspruch 1, wobei das Lufteinblasbauteil (10K) des Abführendes (7K) eine erste Luftkammer (15K) und einen ersten Lufteinblaskanal (16K) aufweist, der die erste Luftkammer (15K) und den Hauptabführkanal (8K) des Abführendes (7K) fluidtechnisch verbindet, wobei der erste Lufteinblaskanal (16K) so angeordnet ist, dass er einen ersten eingeblasenen Luftstrom (F₁K) in einer Richtung zurück in die Abführdüse (6K) bereitstellt.

3. Zyklonabscheidersystem nach Anspruch 2, wobei der erste Lufteinblaskanal (16K) in einem ersten Einblaswinkel (α₁K) angeordnet ist, der kleiner als 60° Grad in Bezug auf eine Längsachse (LK) der Abführdüse (6K) ist, vorzugsweise, wobei der erste Einblaswinkel (α₁K) zwischen 40° und 60° Grad in Bezug auf die Längsachse (LK) der Abführdüse (6K) liegt, z.B. wobei der erste Einblaswinkel (α₁K) etwa 45° Grad beträgt.

4. Zyklonabscheidersystem nach Anspruch 3, wobei die Abführdüse (6K) einen ersten Innenwandabschnitt (17K) aufweist, der in einem ersten Wandwinkel (β₁K) in Bezug auf die Längsachse (LK) der Abführdüse (6K) angeordnet ist, und wobei der erste Einblaswinkel (α₁K) des ersten Lufteinblaskanals (16) im Wesentlichen mit dem ersten Wandwinkel (β₁K) fluchtet/gleich diesem ist.

5. Zyklonabscheidersystem nach einem der Ansprüche 2 bis 4, wobei der erste Lufteinblaskanal (16K) ein schlitzförmiger Kanal ist, der sich in seitlicher Richtung zwischen der ersten Luftkammer (15K) und einem ersten Abführkanalwandabschnitt (18K) des Hauptabführkanals (8K) erstreckt, vorzugsweise, wobei der schlitzförmige erste Lufteinblaskanal (16K) eine Breite (W₁K) von etwa 0.2 mm bis 1 mm hat, besonders bevorzugt, wobei der schlitzförmige erste Lufteinblaskanal (16K) eine Länge (LsK) von mehr als 50% einer Breite (WcK) des Hauptabführkanals (8K), z.B. mehr als 75%, z.B. mehr als 90%, z.B. mehr als 95%, z.B. 100% der Breite (WcK) hat.

6. Zyklonabscheidersystem nach einem der Ansprüche 2 bis 5, wobei das Lufteinblasbauteil (10K) des Abführendes (7K) eine zweite Luftkammer (19K) und einen zweiten Lufteinblaskanal (20K) aufweist, der die zweite Luftkammer (19K) und den Hauptabführkanal (8K) des Abführendes (7K) fluidtechnisch verbindet, wobei der zweite Lufteinblaskanal (20K) so angeordnet ist, dass er einen zweiten eingeblasenen Luftstrom (F₂K) in einer Richtung zurück in die Abführdüse (6K) bereitstellt.

7. Zyklonabscheidersystem nach Anspruch 6, wobei der zweite Lufteinblaskanal (20K) in einem zweiten Einblaswinkel (α₂K) kleiner als 60° in Bezug auf eine Längsachse (LK) der Abführdüse (6K) angeordnet ist.

8. Zyklonabscheidersystem nach Anspruch 7, wobei der zweite Einblaswinkel (α₂K) zwischen 40° und 60° in Bezug auf die Längsachse (LK) der Abführdüse (6K) liegt, z.B. wobei der zweite Einblaswinkel (α₂K) etwa 45° beträgt.

9. Zyklonabscheidersystem nach Anspruch 7, wobei die Abführdüse (6K) einen zweiten Innenwandabschnitt (21K) aufweist, der in einem zweiten Wandwinkel (β₂K) in Bezug auf die Längsachse (LK) der Abführdüse (6K) angeordnet ist, und wobei der zweite Einblaswinkel (α₂K) des zweiten Lufteinblaskanals (20K) im Wesentlichen mit dem zweiten Wandwinkel (β₂K) ausgerichtet ist.

10. Zyklonabscheidersystem nach einem der Ansprüche 6 bis 9, wobei der zweite Lufteinblaskanal (20K) ein schlitzförmiger Kanal ist, der sich zwischen der zweiten Luftkammer (19K) und einem zweiten Abführkanalwandabschnitt (22K) des Hauptabführkanals (8K) erstreckt, vorzugsweise, wobei der schlitzförmige zweite Lufteinblaskanal (20K) eine Breite (W₂K) von etwa 0.2 mm bis 1 mm hat.

11. Zyklonabscheidersystem nach einem der Ansprüche 6 bis 10, wobei der erste und zweite Lufteinblaskanal (16K, 20K) auf gegenüberliegenden Seiten des Hauptabführkanals (8K) angeordnet sind.

12. Zyklonabscheidersystem nach Anspruch 11, wobei die ersten und zweiten Lufteinblaskanäle (16K, 20K) seitlich/seitwärts in entgegengesetzter Richtung beabstandet sind, insbesondere, wenn von den Ansprüchen 5 und 10 abhängend, wobei die schlitzförmigen ersten und zweiten Lufteinblaskanäle (16K, 20K) jeweils eine Länge (LsK) von höchstens 50% einer Breite (WcK) des Hauptabführkanals (8K) haben.

13. Zyklonabscheidersystem nach Anspruch 11, wobei der erste Lufteinblaskanal (16K) eine Mehrzahl von ersten Kanalabschnitten (16aK) aufweist und wobei der zweite Lufteinblaskanal (20K) eine Mehrzahl von zweiten Kanalabschnitten (20aK) aufweist, wobei die Mehrzahl von ersten und zweiten Kanalabschnitten (16aK, 20aK) seitlich/seitwärts in alternierender Weise entlang einer Breite (WcK) des Hauptabführkanals (8K) versetzt angeordnet sind.

14. Zyklonabscheidersystem nach einem der Ansprüche 1 bis 13, wobei, wenn abhängend von Anspruch 2 und 8, die erste und die zweite Luftkammer (15K, 19K) auf gegenüberliegenden Seiten des Hauptabführkanals (8K) angeordnet sind und fluidtechnisch miteinander verbunden sind.

15. Zyklonabscheidersystem nach einem der Ansprüche 1 bis 14, wobei ein Einlassende (12K) der Abführdüse (6K) kreisförmig ist und wobei der Hauptabführkanal (8K) der Abführdüse (6K) im Wesentlichen rechteckig ist, vorzugsweise wobei, wenn abhängend von Anspruch 2, das Lufteinblasbauteil (10K) des Abführendes (7K) zwei gegenüberliegende Hilfsluftkammern (30K) und zwei gegenüberliegende Hilfseinblaskanäle (29K) aufweist, von denen jeder eine der beiden Hilfsluftkammern (30K) mit dem Hauptabführkanal (8K) des Abführendes (7K) fluidtechnisch verbindet,
wobei jeder Hilfseinblaskanal (29K) angeordnet ist, um einen Hilfseinblasluftstrom (G1K, G2K) in einer Richtung zurück in die Abführdüse (6K) bereitzustellen, und
wobei die zwei gegenüberliegenden Hilfsluftkammern (30K) und die zwei gegenüberliegenden Hilfseinblaskanäle (29K) entlang der kürzesten Seiten (SsK) des im Wesentlichen rechteckig geformten Hauptabführkanals (8K) angeordnet sind.

16. Zyklonabscheidersystem nach einem der Ansprüche 1 bis 15, das ferner ein kamerabasiertes Zählsystem (23K) aufweist, das vorzugsweise am Abführende (7K) der Abführdüse (6K) angeordnet ist, wobei das kameragestützte Zählsystem (23K) ein ebenes dreieckiges Sichtfeld (FVK) definiert und wobei der Hauptabführkanal (8K) einen trapezförmigen Querschnitt mit zwei gegenüberliegenden nicht parallelen Seiten (25K, 26K) aufweist, von denen jede parallel zu einer Kante (27K) des ebenen dreieckigen Sichtfeldes (FVK) ist.

17. Zyklonabscheidersystem nach einem der Ansprüche 1 bis 16, wobei jeder der Einlasskanäle (5K) eine Luftverstärkereinheit (5aK) aufweist, um dem Luftstrom (AK) in einer Strömungsrichtung einen zusätzlichen Luftstrom bereitzustellen.

18. Zyklonabscheidersystem nach einem der Ansprüche 1 bis 17, wobei die Abführdüse (6K) ferner ein Abführleitbauteil (32K) aufweist, das an dem Abführende (7K) der Abführdüse (6K) montiert ist,
wobei das Abführleitbauteil (32K) einen expandierenden Leitkanal (33K) aufweist, der fluidtechnisch an den Hauptabführkanal (8K) gekoppelt ist, um lebende Insekten aufzunehmen, wenn das Zyklonabscheidersystem (1K) in Betrieb ist,
vorzugsweise, wobei die Abführdüse (6K) eine sich seitlich erstreckende, längliche Öffnung (34K) aufweist, die zwischen dem Abführleitbauteil (32K) und dem Abführende (7K) angeordnet ist, und
wobei die sich seitlich erstreckende längliche Öffnung (34K) sich ferner parallel entlang des Hauptabführkanals (8K) und entlang einer Öffnungsbreite (WoK) erstreckt, die gleich oder größer ist als eine Breite (WcK) des Hauptabführkanals (8K),
noch bevorzugter, wobei das Abführleitbauteil (32K) ferner einen unteren Umfangsrandabschnitt (35K), z.B. einen Umfangsflanschabschnitt, aufweisen kann, der zum Eingriff mit einem Umfangsrandabschnitt (24aK) eines Behälters (24K) gestaltet ist.

19. Verfahren zum Bereitstellen von Losgrößen lebender Insekten, das aufweist
a) Bereitstellen eines Zyklonabscheidersystems (1K) nach einem der Ansprüche 1 bis 18;
b) Verbinden jedes der einen oder mehreren Einlasskanäle (5K) mit einer Primärluftquelle, die einen Luftstrom (AK) bereitstellt, der lebende Insekten aufweist, und Verbinden des Lufteinblasbauteils (10K) mit einer Sekundärluftquelle;
c) Sammeln der abgeschiedenen lebenden Insekten, die aus der Abführdüse (6K) abgeführt werden; und
wenn eine gewünschte Anzahl von lebenden Insekten gesammelt worden ist,
d) Einblasen von Luft zurück in die Abführdüse (6K) mit dem Lufteinblasbauteil (10K) für eine vorbestimmte Zeitspanne, um das Abführen von lebenden Insekten aus der Abführdüse (6K) vorübergehend zu unterbinden; und
während der vorbestimmten Zeitspanne,
e) Transferieren der gesammelten lebenden Insekten weg von der Abführdüse (6K).

## Revendications

1. Système de séparation à cyclone (1K) destiné à séparer des insectes vivants transportés par un flux d'air, comprenant :
une chambre cyclonique principale (2K) présentant une partie de chambre supérieure (3K) et une partie de chambre inférieure de forme conique (4K), dans lequel la partie de chambre supérieure (3K) est reliée à un ou plusieurs canaux d'admission (5K) qui sont chacun agencés pour être reliés à une source d'air primaire fournissant un flux d'air (AK) comprenant des insectes vivants, et dans lequel la partie de chambre inférieure (4K) est reliée à une buse de décharge (6K) comprenant une extrémité de décharge (7K) présentant un conduit de décharge principal (8K) pour décharger les insectes vivants à partir du système de séparation à cyclone (1K), et
**caractérisé en ce que**
l'extrémité de décharge (7K) comprend un élément d'injection d'air (10K) destiné à être relié à une source d'air secondaire et dans lequel l'élément d'injection d'air (10K) est configuré pour injecter de l'air en retour dans la buse de décharge (6K) afin d'arrêter la décharge des insectes vivants séparés.

2. Système de séparation à cyclone selon la revendication 1, dans lequel l'élément d'injection d'air (10K) de l'extrémité de décharge (7K) comprend une première chambre d'air (15K) et un premier conduit d'injection d'air (16K) reliant de manière fluidique la première chambre d'air (15K) et le conduit de décharge principal (8K) de l'extrémité de décharge (7K), dans lequel le premier conduit d'injection d'air (16K) est agencé pour fournir un premier écoulement d'air injecté (F1K) dans une direction de retour dans la buse de décharge (6K).

3. Système de séparation à cyclone selon la revendication 2, dans lequel le premier conduit d'injection d'air (16K) est agencé à un premier angle d'injection (α₁K) inférieur à 60° degrés par rapport à un axe longitudinal (LK) de la buse de décharge (6K),
de préférence, dans lequel le premier angle d'injection (α₁K) se situe entre 40° et 60° degrés par rapport à l'axe longitudinal (LK) de la buse de décharge (6K), par exemple dans lequel le premier angle d'injection (α₁K) est d'environ 45° degrés.

4. Système de séparation à cyclone selon la revendication 3, dans lequel la buse de décharge (6K) comprend une première partie de paroi intérieure (17K) qui est agencée à un premier angle de paroi (β₁K) par rapport à l'axe longitudinal (LK) de la buse de décharge (6K), et dans lequel le premier angle d'injection (α₁K) du premier conduit d'injection d'air (16) est sensiblement aligné avec/égal au premier angle de paroi (β₁K).

5. Système de séparation à cyclone selon l'une quelconque des revendications 2 à 4, dans lequel le premier conduit d'injection d'air (16K) est un conduit en forme de fente s'étendant dans une direction latérale entre la première chambre d'air (15K) et une première partie de paroi de conduit de décharge (18K) du conduit de décharge principal (8K),
de préférence, dans lequel le premier conduit d'injection d'air en forme de fente (16K) présente une largeur (W₁K) d'environ 0,2 mm à 1 mm, de manière plus préférée dans lequel le premier conduit d'injection d'air en forme de fente (16K) présente une longueur (LsK) supérieure à 50 % d'une largeur (WcK) du conduit de décharge principal (8K), par exemple supérieure à 75 %, par exemple supérieure à 90 %, par exemple supérieure à 95 %, par exemple égale à 100 % de la largeur (WcK).

6. Système de séparation à cyclone selon l'une quelconque des revendications 2 à 5, dans lequel l'élément d'injection d'air (10K) de l'extrémité de décharge (7K) comprend une seconde chambre d'air (19K) et un second conduit d'injection d'air (20K) reliant de manière fluidique la seconde chambre d'air (19K) et le conduit de décharge principal (8K) de l'extrémité de décharge (7K), dans lequel le second conduit d'injection d'air (20K) est agencé pour fournir un second écoulement d'air injecté (F2K) dans une direction de retour dans la buse de décharge (6K).

7. Système de séparation à cyclone selon la revendication 6, dans lequel le second conduit d'injection d'air (20K) est agencé à un second angle d'injection (α₂K) inférieur à 60° degrés par rapport à un axe longitudinal (LK) de la buse de décharge (6K).

8. Système de séparation à cyclone selon la revendication 7, dans lequel le second angle d'injection (α₂K) se situe entre 40° et 60° degrés par rapport à l'axe longitudinal (LK) de la buse de décharge (6K), par exemple dans lequel le second angle d'injection (α₂K) est d'environ 45° degrés.

9. Système de séparation à cyclone selon la revendication 7, dans lequel la buse de décharge (6K) comprend une seconde partie de paroi intérieure (21K) étant agencée à un second angle de paroi (β₂K) par rapport à l'axe longitudinal (LK) de la buse de décharge (6K), et dans lequel le second angle d'injection (α₂K) du second conduit d'injection d'air (20K) est sensiblement aligné avec le second angle de paroi (β₂K).

10. Système de séparation à cyclone selon l'une quelconque des revendications 6 à 9, dans lequel le second conduit d'injection d'air (20K) est un conduit en forme de fente s'étendant entre la seconde chambre d'air (19K) et une seconde partie de paroi de conduit de décharge (22K) du conduit de décharge principal (8K), de préférence dans lequel le second conduit d'injection d'air en forme de fente (20K) présente une largeur (W₂K) d'environ 0,2 mm à 1 mm.

11. Système de séparation à cyclone selon l'une quelconque des revendications 6 à 10, dans lequel les premier et second conduits d'injection d'air (16K, 20K) sont agencés sur des côtés opposés du conduit de décharge principal (8K).

12. Système de séparation à cyclone selon la revendication 11, dans lequel les premier et second conduits d'injection d'air (16K, 20K) sont décalés transversalement/latéralement dans une direction opposée, de préférence lorsque dépendant es revendications 5 et 10, dans lequel les premier et second conduits d'injection d'air en forme de fente (16K, 20K) présentent chacun une longueur (LsK) d'au plus 50 % d'une largeur (WcK) du conduit de décharge principal (8K).

13. Système de séparation à cyclone selon la revendication 11, dans lequel le premier conduit d'injection d'air (16K) comprend une pluralité de premières sections de conduit (16aK) et dans lequel le second conduit d'injection d'air (20K) comprend une pluralité de secondes sections de conduit (20aK), dans lequel la pluralité de premières et secondes sections de conduit (16aK, 20aK) sont décalées transversalement/latéralement de manière alternée le long d'une largeur (WcK) du conduit de décharge principal (8K).

14. Système de séparation à cyclone selon l'une quelconque des revendications 1 à 13, lorsque dépendant des revendications 2 et 8, dans lequel les première et seconde chambres d'air (15K, 19K) sont agencées sur des côtés opposés du conduit de décharge principal (8K) et sont reliées de manière fluidique l'une à l'autre.

15. Système de séparation à cyclone selon l'une quelconque des revendications 1 à 14, dans lequel une extrémité d'admission (12K) de la buse de décharge (6K) est circulaire et dans lequel le conduit de décharge principal (8K) de la buse de décharge (6K) est sensiblement rectangulaire,
de préférence, lorsque dépendant de la revendication 2, dans lequel l'élément d'injection d'air (10K) de l'extrémité de décharge (7K) comprend deux chambres d'air auxiliaires opposées (30K) et deux conduits d'injection auxiliaires opposés (29K) dont chacun relie de manière fluidique l'une des deux chambres d'air auxiliaires (30K) au conduit de décharge principal (8K) de l'extrémité de décharge (7K),
dans lequel chaque conduit d'injection auxiliaire (29K) est agencé pour fournir un flux d'air injecté auxiliaire (G1K, G2K) dans une direction de retour dans la buse de décharge (6K), et
dans lequel les deux chambres d'air auxiliaires opposées (30K) et les deux conduits d'injection auxiliaires opposés (29K) sont agencés le long des côtés les plus courts (SsK) du conduit de décharge principal (8K) de forme sensiblement rectangulaire.

16. Système de séparation à cyclone selon l'une quelconque des revendications 1 à 15, comprenant en outre un système de comptage à base de caméra (23k) agencé à l'extrémité de décharge (7K) de la buse de décharge (6K),
de préférence, dans lequel le système de comptage à base de caméra (23K) définit un champ de vision triangulaire plan (FVK) et dans lequel le conduit de décharge principal (8K) comprend une section transversale en forme de trapèze présentant deux côtés opposés non parallèles (25K, 26K) dont chacun est parallèle à un bord (27K) du champ de vision triangulaire plan (FVK).

17. Système de séparation à cyclone selon l'une quelconque des revendications 1 à 16, dans lequel chacun des canaux d'admission (5K) comprend une unité d'amplificateur d'air (5aK) pour fournir un flux d'air supplémentaire au flux d'air (AK) dans une direction d'écoulement de celui-ci.

18. Système de séparation à cyclone selon l'une quelconque des revendications 1 à 17, dans lequel la buse de décharge (6K) comprend en outre un élément de guidage de décharge (32K) monté sur l'extrémité de décharge (7K) de la buse de décharge (6K),
dans lequel l'élément de guidage de décharge (32K) comprend un canal de guidage expansible (33K) couplé de manière fluidique au conduit de décharge principal (8K) pour recevoir des insectes vivants lorsque le système de séparation à cyclone (1K) est en fonctionnement,
de préférence, dans lequel la buse de décharge (6K) comprend une ouverture allongée s'étendant latéralement (34K) agencée entre l'élément de guidage de décharge (32K) et l'extrémité de décharge (7K), et
dans lequel l'ouverture allongée s'étendant latéralement (34K) s'étend en outre parallèlement le long du conduit de décharge principal (8K) et le long d'une largeur d'ouverture (WoK) égale ou supérieure à une largeur (WcK) du conduit de décharge principal (8K),
de manière plus préférée, dans lequel l'élément de guidage de décharge (32K) peut en outre comprendre une partie de rebord circonférentielle inférieure (35K), par exemple une partie de bride circonférentielle, configurée pour venir en prise avec une partie de rebord circonférentielle (24aK) d'un récipient (24K).

19. Procédé pour fournir des lots d'insectes vivants, comprenant les étapes consistant à
a) fournir un système de séparation à cyclone (1K) selon l'une quelconque des revendications 1 à 18;
b) relier chacun des un ou plusieurs canaux d'admission (5K) à une source d'air primaire fournissant un flux d'air (AK) comprenant des insectes vivants et relier l'élément d'injection d'air (10K) à une source d'air secondaire;
c) collecter les insectes vivants séparés étant déchargés à partir de la buse de décharge (6K) ; et lorsqu'un nombre souhaité d'insectes vivants ont été collectés,
d) réinjecter de l'air dans la buse de décharge (6K) avec l'élément d'injection d'air (10K) pendant une période de temps prédéterminée pour arrêter temporairement la décharge d'insectes vivants à partir de la buse de décharge (6K) ; et pendant la période de temps prédéterminée,
e) transférer les insectes vivants collectés loin de la buse de décharge (6K).
